(19) Europäisches Patentamt / European Patent Office / Office européen des brevets

(11) **EP 2 846 814 B1**

(12) **EUROPEAN PATENT SPECIFICATION**

(45) Date of publication and mention
of the grant of the patent:
**29.06.2016 Bulletin 2016/26**

(21) Application number: **13721599.2**

(22) Date of filing: **08.05.2013**

(51) Int Cl.:
*A61K 36/185* (2006.01)     *A61K 36/53* (2006.01)
*A61K 36/30* (2006.01)       *A61P 31/14* (2006.01)
*A61P 31/18* (2006.01)

(86) International application number:
**PCT/EP2013/001378**

(87) International publication number:
**WO 2013/167275 (14.11.2013 Gazette 2013/46)**

(54) **COMPOSITION FOR THE PREVENTION AND TREATMENT OF VIRAL INFECTIONS CAUSED BY RETROVIRUSES**

ZUSAMMENSETZUNG ZUR VORBEUGUNG UND BEHANDLUNG VON VIRALEN INFEKTIONEN DURCH RETROVIREN

COMPOSITION POUR LA PRÉVENTION ET LE TRAITEMENT D'INFECTIONS VIRALES PROVOQUÉES PAR DES RÉTROVIRUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.05.2012 EP 12003632**
**09.05.2012 US 201261644603 P**

(43) Date of publication of application:
**18.03.2015 Bulletin 2015/12**

(73) Proprietor: **Pandalis, Georgios**
**49219 Glandorf (DE)**

(72) Inventor: **Pandalis, Georgios**
**49219 Glandorf (DE)**

(74) Representative: **Grünecker Patent- und**
**Rechtsanwälte**
**PartG mbB**
**Leopoldstraße 4**
**80802 München (DE)**

(56) References cited:
**EP-A1- 1 837 029          WO-A2-2006/079109**
**WO-A2-2008/032212      WO-A2-2010/066346**

• **SANCHEZ-ARREOLA E ET AL: "TRADITIONAL USES AND A PRELIMINARY STUDY ON THE PHYTOCHEMISTRY OF NINE MEDICINAL PLANT SPECIES OF COMMON USE IN HIGH LAND OF PUEBLA AND TLAXCALA, MEXICO", CROP RESEARCH, AGRICULTURAL RESEARCH INFORMATION CENTRE, HISAR, IN, vol. 30, no. 1, 1 July 2005 (2005-07-01), pages 113-118, XP008080143, ISSN: 0970-4884**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a composition for use in the prophylaxis and/or treatment of viral infections caused by retroviruses.

A great number of human diseases are caused by viruses and include influenza, the common cold, chickenpox and cold sores. Diseases like AIDS (acquired immunodeficiency syndrome), hepatitis, herpes infections, Coxsackie infections, measles, rubella, cytomegaly, mumps, rabies, diarrhea, SARS, Ebola, Yellow fever, West-Nile fever, Hanta fever, Dengue fever, Marburg fever, Lassa fever, smallpox, human papillomaviral infections, infectious mononucleosis, Burkitt lymphoma, polyomyelitis, encephalitis, adenopharyngitis are also virus-borne.

A retrovirus is an RNA virus which uses the enzyme reverse transcriptase to produce DNA from its RNA genome. Reverse transcriptase is a DNA polymerase enzyme that transcribes single-stranded RNA into single-stranded DNA. Further, reverse transcriptase helps in the formation of a double helix DNA once the RNA has been reverse transcribed into a single-strand DNA. The so formed DNA of the virus is then incorporated into the hosts genome by an integrase enzyme and replicated with it. Retroviruses are enveloped viruses belonging to the family of *Retroviridae* and can be divided in endogenous and exogenous retroviruses.

An endogenous retrovirus is present as a genetic element in the chromosomal DNA. It is derived from ancient viral infections in humans, mammals and other vertebrates and passed on to the next generation and remains in the genome. Human endogenous retroviruses are suspected to play a role in some autoimmune diseases, in particular multiple sclerosis.

Exogenous retroviruses are horizontally-transmitted infectious RNA-containing viruses which are transmitted from animal to animal or person to person. Horizontal transmission occurs almost exclusively by body fluids, transmission by smear infections is extremely rare and transmission via air can be excluded. Diseases induced by or associated with exogenous retroviruses are, e. g., feline leukemia or sarcomas, chicken leukemia or sarcomas, mouse leukemia or sarcomas, equine infectious anemia, bovine leukemia, caprine arthritis-encephalitis, human adult T-cell leukemia, human tropic spastic paraparesis, and AIDS.

However, one of the most serious viral infections in humans is an HIV (human immunodeficiency virus) infection. HIV infection in humans has become pandemic. Most HIV infected individuals eventually develop AIDS, a condition in humans in which the immune system begins to fail, leading to life-threatening opportunistic infections. Since 1981 when AIDS was first recognized it has killed more than 25 million people. In 2007 between 30.6 and 36.1 million people were believed to live with HIV, approximately 2.1 million people died and 2.5 million new infections were reported. In Africa, having the highest prevalence of HIV, the average life expectancy is approximately 6.5 years less than it would be without the disease. This leads to a great economical loss and increased poverty.

An HIV infection can be divided into four stages: Primary infection, clinically asymptomatic stage, symptomatic HIV infection and progression from HIV to AIDS.

The first stage of infection, the primary HIV infection, lasts for a few weeks and can include flue-like symptoms. In this stage, the immune system begins to produce HIV antibodies and cytotoxic lymphocytes due to a large amount of HIV in the peripheral blood and lymphatic tissues.

In stage 2, the clinically asymptomatic stage or latency stage, the number of viral particles in the peripheral blood is reduced by strong immune defense. Said stage lasts for approximately 10 years and is free from major symptoms. However, HIV is active in the lymph nodes and people remain infectious.

Stage 3 of the infection, the symptomatic HIV infection, characterizes the stage wherein the immune system becomes highly damaged by HIV. The lymph nodes and tissues become damaged due to the years of activity, HIV mutates and becomes more pathogenic, leading to a higher number of T helper cell destruction, and the infected body fails to keep up with replacing the T helper cells that are lost. Due to the decline in T helper cell numbers, cell mediated immunity is lost, and a variety of opportunistic infections appear. This stage eventually leads to stage 4 of the infection, namely the progression to AIDS.

Vaccination represents the most important means of preventing viral infections. However, currently no vaccine or cure for HIV infection is available.

Generally, infections caused by retroviruses are treated with antiretroviral drugs. HIV infections are currently treated by highly active antiretroviral therapy (HAART). Said therapy is a combination of at least three different drugs belonging to at least two classes of antiretroviral agents. If only one drug was taken, HIV would become resistant to said drug. Taking several antiretroviral drugs at the same time reduces the rate of resistance development, making treatment more effective in the long term. Currently, more than 20 approved antiretroviral drugs divided into 5 groups are available. Each of these groups attacks HIV in a different way. A first group of antiretroviral agents are nucleoside/nucleotide reverse transcriptase inhibitors (NRTI). Said inhibitors interfere with the reverse transcriptase protein which is required by the virus to make new copies of itself. A second group of compounds are non-nucleoside reverse transcriptase inhibitors (NNRTI), which hinder HIV from replication by inhibiting the reverse transcriptase protein. A third group of antiretroviral agents are protease inhibitors (PI). Said agents inhibit protease, which is required in the HIV replication process. A fourth class of drugs are fusion or entry inhibitors, which prevent HIV from binding to or entering human immune cells. Said drugs are particularly used for patients who are infected with viruses already resistant to common therapies. A fifth class of inhibitors are integrase inhibitors which interfere with integrase. Said enzyme is needed for the virus to insert its genetic material into human cells.

Highly active antiretroviral therapy does not cure the patient, but improves the general health and quality of life of HIV infected patients. The average life expectancy of an HIV infected person now is approximately 32 years from the time of infection. In the absence of HAART, progressions of HIV infection to AIDS usually occurs after 9 to 10 years and the median survival time after developing AIDS is only 9 months. The development of HAART as effective therapy for HIV infection has substantially reduced the death rate in those areas where the drugs are widely available.

The most common drug combination in highly active antiretroviral therapy consists of two NRTIs combined with either an NNRTI or a protease inhibitor. Most commonly, ritonavir is used as the protease inhibitor. An example of an antiretroviral drug combination contains two NRTIs, namely zidovudine and lamivudine, in combination with the NNRTI efavirenz. Commonly used NRTIs are lamivudine, abacavir, zidovudine, stavudine, zalcitabine, didanosine, emtricitabine and tenofovir. Typically used NNRTIs are delavirdine, efavirenz, etravirine and nevirapine. Standard protease inhibitors are amprenavir, fosamprenavir, atazanavir, darunavir, indinavir, lopinavir, ritonavir, nelfinavir, saquinavir and tipranavir. As fusion or entry inhibitors enfuvirtide and maraviroc are normally used. A commonly used integrase inhibitor is raltegravir.

However, sometimes patients show a medication intolerance and highly active antiretroviral therapy in some cases causes serious side effects, which can even be life-threatening in rare cases. Further, non-adherence and non-persistence are the major reasons for a failing therapy. Reasons for non-adherence and non-persistence are psychosocial issues, e. g. poor access to medical support, inadequate social support, psychiatric disease and drug abuse. Further, the regimens are very complex, requiring a great number of pills, specific dosing frequency, meal restrictions and other issues. Typical side effects which occur in the therapy are lipodystrophy, dyslipidemia, insulin resistance, an increase in cardiovascular risks and birth defects. Further side effects which occur during treatment with antiretroviral drugs are diarrhoea, nausea, vomiting, rash, hypersensitivity reactions, appetite loss, central nervous system effects, such as dizziness, mood changes, depression, anxiety, and paranoia, fatigue, insomnia, kidney damage, liver damage, pancreas damage, lactic acidosis and nerve damage. Said side effects can have a major impact on health or quality of life.

Moreover, antiretroviral drugs are expensive and the majority of the infected individuals does not have access to medications and treatments for HIV and AIDS. For example, fusion and entry inhibitors, as well as integrase inhibitors are only available in resource-rich countries.

Another therapeutic form for treating diseases is phytotherapy. In phytotherapy plants and plant parts are used for treating the disease. The plants or plant parts are administered in form of teas, juices, tinctures, powders, decoctions and extracts.

Kashman et al., Journal of Medicinal Chemistry 1992, 35 (15), pages 2735 to 2743 and Dharmaratne et al., Planta Medica 1998, 64 (5), pages 460 to 461 isolated compounds from *Calophyllum lanigerum* and *Calophyllum cordato-oblongum*, which showed antiviral activity against HIV-1.

Hu et al., Journal of Natural Products 1994, 57 (1), pages 42 to 51 isolated compounds from a methanolic extract of the flower heads of *Chrysanthemum morifolium.* One of the isolated compounds, acacetin-7-O-β-D-galactopyranoside, had significant anti-HIV activity.

Silvia Geuenich, "Untersuchungen zum Pathogenitätspotential von HIV-1 Primärisolaten und mechanistische Analysen zur antiviralen Aktivitat wassriger Extrakte und Flavonoide aus Lamiaceen", Inaugural Dissertation, Heidelberg, 2008, studied the anti-HIV-1 activity of isolates of aqueous extracts of *Melissa officinalis*, *Mentha* x *piperita*, and *Salvia officinalis*. She furthermore isolated two flavones from an extract of *Marrubium peregrinum,* which both showed an anti-HIV-1 activity. However, all of the extracts were further concentrated and the particular compounds were isolated.

The object of the present invention is to provide an antiviral composition for the prophylaxis and/or treatment of viral infections caused by retroviruses, which can be economically prepared and which causes no side-effects at all or only minor side-effects when administered.

This object is achieved by a composition for use in the prophylaxis and/or treatment of viral infections caused by retroviruses, comprising a concentrate or an extract of marrubium in combination with at least one concentrate or extract of another plant selected from *Ribes nigrum, Cistus incanus*, and *Borago officinalis*. Preferred embodiments are described in the subclaims 2 to 11.

Description of the figures

Figure 1 shows the results of the antiviral activity of an extract of *Marrubium vulgare* and *Ribes nigrum* against HIV-1 (single extracts and combination).

Figure 2 shows the results of the antiviral activity of an extract of *Marrubium vulgare* and *Cistus incanus* against HIV-1 (single extracts and combination).

Figure 3 shows the results of the antiviral activity of an extract of *Marrubium vulgare* and *Borago officinalis* against HIV-1 (single extracts and combination).

Figure 4 shows the results of the cytotoxicity assay of a combination of an extract of *Marrubium vulgare* and *Borago officinalis*.

Figure 5 shows the results of the cytotoxicity assay of an extract of the twigs and leaves of *Marrubium vulgare* and *Ribes nigrum,* and *Marrubium vulgare* and *Cistus incanus* (single extracts and combinations).

Detailed description of the invention

The present invention relates to a composition for the use in the prophylaxis and/or treatment of viral infections caused by retroviruses, wherein the composition comprises a concentrate or an extract of a plant of the genus marrubium in combination with at least one concentrate or extract of another plant selected from *Ribes nigrum, Cistus incanus*, and *Borago officinalis*.

In a preferred embodiment in combination with any one of the embodiments listed above or below, the composition according to the invention comprises a concentrate or an extract of a plant of the genus marrubium and a concentrate or extract of another plant in a ratio of 1 : 10 to 10 : 1, preferably 1 : 5 to 5 : 1, and more preferably 1 : 2 to 2 : 1, and in particular 1 : 1.

In a preferred embodiment in combination with any one of the embodiments listed above or below, the concentrate or extract is from the aboveground parts of the plant, more preferably the aboveground parts are selected from the group consisting of leaves and twigs.

In another preferred embodiment in combination with any one of the embodiments listed above or below, the concentrate or extract of marrubium is from *Marrubium vulgare*.

In a further preferred embodiment in combination with any one of the embodiments listed above or below, the composition comprises two or more concentrates or extracts of the other plant. Particularly preferred embodiments are *Marrubium vulgare*/*Cistus incanus*/*Ribes nigrum*, *Marrubium vulgare*/*Cistus incanus*/*Borago officinalis*, and *Marrubium vulgare*/*Ribes nigrum*/*Borago officinalis*.

In a preferred embodiment in combination with any one of the embodiments listed above or below, the viral infection is caused by exogenous retroviruses, more preferably the viral infection is caused by lentiviruses. In particular, the viral infection is caused by HIV-1 and/or HIV-2.

In a further preferred embodiment in combination with any one of the embodiments listed above or below, the composition is in liquid, dry or semi-solid form.

In a preferred embodiment in combination with any one of the embodiments listed above or below, the extract is an aqueous extract or an alcoholic extract.

In a preferred embodiment in combination with any one of the embodiments listed above or below, the composition is administered orally, topically, rectally, or vaginally, more preferably, the composition is administered orally, rectally, or vaginally.

In a further preferred embodiment in combination with any one of the embodiments listed above or below, the composition is in the form of an aerosol, mouthwash, or mouth spray. The composition may also preferably be present in the form of a tablet, coated tablet, effervescent tablet, capsule, powder, granulate, sugar-coated tablet, lozenge, pill, ampoule, drop, suppository, emulsion, ointment, gel, tinctures, paste, cream, moist compress, gargling solution or plant juice. More preferably, the composition is in the form of a mouth spray, mouthwash, suppository, cream or ointment, in particular in the form of a cream or ointment for rectal of vaginal administration.

In the present invention the term "aboveground parts of a plant" refers to all parts which are aboveground, including leaves, twigs, blossoms, fruits and seeds. For preparing the concentrate or extract used according to the present invention, preferably the leaves and twigs are used.

In the present invention, the term "twig" refers to a small shoot or branch having a diameter of 3 cm at most. Preferably, the diameter of the twigs is up to 1 cm.

According to the invention, the term "extract" is used representatively for all products that are obtained from a herbal subject by means of an extraction with a solvent, such as with maceration or percolation. The term "concentrate" is used representatively for all products that are obtained from a herbal subject by means of removal of water from the fresh plant. A dry plant product, such as a powder, is also included in the term "concentrate".

Generally, an extraction of the plant parts including leaves, twigs and fruits with a suitable solvent takes place. Suitable solvents are water, alcohols, such as methanol, ethanol or isopropyl alcohol, or chlorinated solvents, such as dichloromethane, as well as acetone, acetylacetone, ethylacetate, ammonia or glacial acetic acid, but also supercritical carbon dioxide. Mixtures of the solvents mentioned can also be used. In a preferred embodiment, in combination with any one of the embodiments listed above or below, water or a mixture of water with methanol or ethanol is used.

The extraction is normally carried out at temperatures of 25 °C to, where applicable, as high as the boiling point of the solvent used. Preferred is an extraction at 95 to 100 °C.

The extraction is normally carried out for 2 to 8 h. Preferably, the extraction is carried out for 3 to 6 h, more preferably for 4 to 5 h. Furthermore, fats, such as pork fat, waxes, such as beeswax, or oils, such as olive oil and almond oil, can be used for the extraction. Preferably, almond oil is used.

In order to achieve the highest possible yield, the plant material can be extracted a number of times. Preferably, the extraction is repeated 2 to 6 times, more preferably 3 times. In this case, it is also possible to use different solvents in the various extraction steps or an extraction with a solvent can be followed by an extraction with a fat, wax or oil, or vice versa.

As a result of the extraction, a liquid, semi-solid or solid raw product is obtained, which can be used in this form for producing a composition for the prophylaxis and/or treatment of viral infections caused by retroviruses.

A maceration procedure is normally performed for five to nine days, preferably for seven days, at room temperature with a mixture of water and ethanol, by pouring the solvent mixture over the plant elements and letting this stand for the period of time mentioned.

According to the invention, a percolation of the plant parts is normally achieved by treating the parts with water at 95 to 100 °C for four to five hours by conducting the water through the plant parts.

The crude product obtained from an extraction with a solvent, such as a maceration or percolation, can also be concentrated and/or dried and/or further processed before use. The further processing can, for example, include cleaning steps known to the person skilled in the art, such as centrifugation, filtration and decanting, in order to remove suspended materials from the extract. Chromatography, such as column chromatography, gas chromatography or HPLC or steam distillation may also be used for purification. In a preferred embodiment the crude product is used without further purification steps.

An extract obtained in this way can subsequently be further processed into a dry extract. To produce the dry extract, the solvent can be withdrawn from the liquid raw extract, the concentrated extract or the cleaned extract by, for example, spray drying, freeze drying or vacuum drying.

In the present invention, the term "prophylaxis" refers to a procedure to prevent an infection. Prophylactic measures can be divided into primary prophylaxis (prevention of the infection) and secondary prophylaxis (protection from the disease).

Virions of retroviruses consist of enveloped particles having a particle diameter of about 100 nm and also contain two identical single-stranded RNA molecules of 7 to 10 kb in length. The main components of retroviruses are

• an envelope composed of a lipid bilayer obtained from the host plasma membrane,

• dimer RNA having a cap at 5' end and polyadenyl at 3' end, and

• proteins containing gag proteins as major components of the viral capsid, protease functioning in proteolytic cleavages during virion maturation, pol proteins responsible for the synthesis of viral DNA and integration into host DNA after infection, and env proteins playing a role in association and entry of virion into the host cell.

HIV is a virus belonging to the family of *Retroviridae* and the genus of lentivirus. It is roughly spherical having a diameter of about 120 nm and is composed of two copies of positive single-stranded RNA, which is bound to nucleocapsid proteins and enzymes required for the replication of the virion (e. g. reverse transcriptase, proteases, ribonuclease and integrase). The capsid is surrounded by a matrix composed of viral protein, which is further surrounded by two layers of phospholipids from the membrane of a human cell. In said membrane further proteins from the host cell and trimeric viral envelope proteins are embedded. These envelope spikes consist of a cap made of three molecules of HIV-1 glycoprotein gp120 and a stem composed of HIV-1 transmembrane protein gp41 that anchors the spike into the lipid bilayer. The viral glycoproteins enable the virus to attach and fuse with target cells to start the infectious cycle.

HIV infects cells of the immune system and the central nervous system. In particular, HIV enters macrophages and T helper cells, in particular CD4+ T cells, by binding of the viral glycoproteins gp120 to receptors on the target cell, fusion of the viral envelope with the cell membrane and release of the HIV capsid into the cell. After penetration into the cell, HIV produces new copies of itself which continue to infect other cells. Thus, HIV infection finally leads to a reduction in the number of T helper cells through three main mechansims by direct viral killing of infected cells, increased rate of apoptosis in infected cells and killing of infected CD4+ T cells by CD8 cytotoxic lymphocytes that recognize infected cells. If the level of CD4+ T cells declines below a critical level, cell-mediated immunity is lost and the body becomes more susceptible to opportunistic infections. When sexually transmitted, HIV is carried by dendritic cells to the proximal lymph nodes, where a massive replication occurs.

HIV can be further divided into HIV-1 and HIV-2. For HIV-1, the subtypes A to J exist, the most common subtypes are 1A, 1B, 1C and 1D. Further more intersubtype recombinants exist, which also contribute to the pandemic (circulating recombinant forms CRF, i.e. in Thailand). HIV-2 can be divided in subtypes A to E. It is less pathogenic than HIV-1.

Besides HIV, another human retrovirus is the human T-lymphotropic virus, in particular human T-lymphotropic virus Type 1 (HTLV-1). HTLV-1 is a human RNA retrovirus that causes T-cell leukemia and T-cell lymphoma and may also be involved in demyelinating diseases, such as tropical spastic paraparesis.

Further retroviruses include Avian leukosis virus, Rous sarcome virus, Mouse mammary tumor virus, Murin leukemia virus, Feline leukemia virus, Bovine leukemia virus, Walley dermal sarcoma virus, Simian and Feline immunodeficency virus, Equine infectious anemia virus and Simian foamy virus causing diseases such as feline leukemia or sarcomas, chicken leukemia or sarcomas, mouse leukemia or sarcomas, equine infectious anemia, bovine leukemia, and caprine arthritis-encephalitis.

According to the invention, an extract or a concentrate of marrubium is used in combination with at least one concentrate or extract of another plant selected from *Ribes nigrum, Cistus incanus*, and *Borago officinalis* for producing a composition for the prophylaxis and/or treatment of viral infections caused by retroviruses.

Marrubium is a genus of the family of *Lamiaceae* of about 40 species of flowering plants native to temperate regions of Europe and Asia.

Of particular interest in the present invention is *Marrubium vulgare*.

*Marubium vulgare* contains traces of essential oils based on monoterpenoid components camphene, p-cymene, fenchene, limonene, $\alpha$-pinen, sabinene, and $\alpha$-terpineol. It further contains labdane diterpenes, such as marrubiin, marrubenol, marrubiol, peregrinol, and vulgarol; tanning agents, such as chlorogenic acid, caffeic acid, caffeoylquinic acid, and cryptochlorogenic acid; and flavonoids, particularly flavonglycosides, flavonolgylcosides, flavons and flavonols, such as apigenin, luteolin, quercetin, vicenin, and vitexin (Hager's Handbuch der pharmazeutischen Praxis, Drogen E-O, 5., vollständig neubearbeitete Auflage, Springer-Verlag, 1993, pages 777-782).

*Ribes nigrum* belongs to the family of *Grossulariaceae* covering about 150 species of flowering plants, which are native throughout the temperate regions of the Northern Hemisphere, and in the mountain ranges of Central and South America.

*Ribes nigrum* contains flavonoids, terpenoids and essential oils in different concentrations. For example, the concentration may differ with respect to the leaves and fruits. Thus, in the following, the leaves and fruits are considered separately.

The scientific term *Ribes nigri folium* refers to the leaves of *Ribes nigrum,* whereas the term *Ribes nigri fructus* refers to the fruits of *Ribes nigrum.*

Some of the components detected in *Ribes nigrum* are flavonols, such as quercetin and myricetin and their glycosides, as well as dimers or oligomers of proanthocyanidins. *Ribes nigri folium* comprises traces of essential oils, flavonolgylcoside and proanthocyanidine. *Ribes nigri fructus* contains anthocyanidins and flavonolglycosides, in particular isoquercitin, myricetin-D-glucopyranosid and rutosin. Further components of *Ribes nigri fructus* are fruit acids, such as citric acid, isocitric acid and malic acid, hydroxycinammic acid derivatives, Vitamin C and in the seeds $\gamma$-linolic acid (Hager's Handbuch der pharmazeutischen Praxis, Drogen P-Z, 5., vollständig neubearbeitete Auflage, Springer-Verlag, 1993, pages 466-474).

In a preferred embodiment, in combination with any one of the embodiments listed above or below, the extract or concentrate of the other plant is from *Ribes nigri folium.*

In another embodiment in combination with one of the embodiments listed above or below, the extract or concentrate of the other plant may be selected from *Cistus incanus* (also known as *Cistus creticus*).

Plants of the genus cistus contain essential oils containing approximately 60 % by weight of diterpenes with manoyl oxide as main component of the diterpenes. They furthermore contain a resin fraction comprising labdane diterpene alcohols and labdane diterpene acids, triterpene acids of the damarane type and fatty acid esters. Further components of cistus are flavonoids such as apigenin, luteolin, quercetin, myricetin and kaempferol, as well as methylether derivatives and glycosides thereof.

In another embodiment in combination with one of the embodiments listed above or below, the extract or concentrate of the other plant may be selected from *Borago officinalis.*

The ingredients of *Borago officinalis* vary widely with respect to the leaves, flowers and seeds of the plant. Thus, in the following, the leaves, flowers and seeds are considered separately.

The seeds mainly contain proteins and oils, in particular $\gamma$-linolenic acid. The flowers of *Borago officinalis*, *Boraginis flos,* contain small amounts of bornesit, allantoin, mucus substances comprising glucose, galactose and arabinose residues, and mineral salts, particularly potassium salts. The leaves of *Borago officinalis*, *Boraginis herba*, contain tanning agents, silicic acid, mucus substances comprising glucose, galactose and arabinose residues, and pyrrolizidine alkaloids (Hager's Handbuch der pharmazeutischen Praxis, Drogen A-D, 5., vollständig neubearbeitete Auflage, Springer-Verlag, 1993, pages 528-532).

The composition for use according to the invention is preferably produced from at least one of the aboveground parts of the plants selected from the group of leaves and twigs. More preferably, the aboveground shoots of the plant that grow back in the same year are used. In general, all elements of the aboveground part of the plant, such as leaves, twigs, blossoms, fruits and seeds can be used. Preferably, the twigs are used with leaves and blossoms.

In a preferred embodiment in combination with any one of the embodiments listed above or below, the composition for use according to the present invention can be produced by either mixing the parts of the plant of marrubium and the other plant before carrying out the extraction; alternatively and preferably, the concentrate or extract of marrubium is mixed with the concentrate or extract of the other plant so as to obtain the composition for use according to the invention.

The parts of the plant, including leaves, twigs and fruits can be either dried or pressed out directly after the harvest, meaning in the raw state, after being broken up, where appropriate, in order to produce a juice from the pressing.

In a further embodiment, in combination with any one of the embodiments listed above or below, the plant parts are submitted in the raw state to an extraction with a solvent, such as a maceration or percolation, for example. Alternatively, the plant parts can also be dried and/or subsequently broken into small pieces in a suitable manner before the extraction, by means of rubbing or cutting them, for example.

The composition for use according to the invention can be used for the prophylaxis and/or treatment of viral infections caused by retroviruses in each of the forms described above. The composition is preferably used for the prophylaxis and/or treatment of viral infections that are caused by lentiviruses, in particular HIV-1 and/or HIV-2.

The composition according to the invention can therefore be administered as a medicine. In addition to therapeutic use, the composition is also suitable for non-therapeutic treatment of viral infections caused by retroviruses.

The composition can be applied in each of the application forms familiar to the person skilled in the art for both medical and non-medical use, e.g., as tablet, coated tablet, effervescent tablet, capsule, powder, granulate, sugar-coated tablet, lozenge, pill, ampoule, drop, suppository, emulsion, ointment, cream, gel, tincture, paste, moist compress, solution or spray. In galenic and other application forms, the composition can be processed with the customary galenic aids, such as tablet bonders, filling agents, preservative agents, tablet-opening agents, flow regulation agents, softening agents, wetting agents, dispersing agents, emulsifying agents, solvents, retarding agents, anti-oxidative agents, consistency regulators, penetration improvers and/or propellant gases. Further elements, such as vitamins and minerals, can be added to the composition used according to the invention.

The concentration of the composition in the application form varies, depending on the type of application. As a rule, the quantity of the composition amounts to between 0.5 and 1,000 mg per dosing unit for solid application forms. Preferably the quantity of the composition amounts to between 1 and 500 mg per unit. In liquid application forms, the composition can be in a concentration of 0.1 $\mu$g/ml to 100 mg/ml, preferably from 1 $\mu$g/ml to 50 mg/ml, more preferably from 10 to 500 $\mu$g/ml, in particular 15 to 100 $\mu$g/ml. In the case of semi-solid application forms, the content of the composition amounts to 1 to 90% by weight, preferably 5 to 75 % by weight.

In a preferred embodiment, in combination with any one of the embodiments listed above or below, the composition is administered in the form of a tablet, coated tablet, effervescent tablet; capsule, powder, granulate, sugar-coated tablet, lozenge, pill, ampoule, drop, for oral application. It is preferable for the composition be in the form of an extract in this case. Most especially preferred, the composition is in the form of a dry extract.

In a further preferred embodiment, in combination with any one of the embodiments listed above or below, the composition is administered in the form of an emulsion, ointment, gel, tincture, paste, cream or moist compress for topical application. For rectal or vaginal application, the composition is administered in the form of an emulsion, ointment, gel, tincture, paste, cream or suppository. In those cases, the composition is preferably used in the form of an extract in which the active substances are withdrawn from the plant by means of extraction with a fat, wax or oil. It is furthermore preferred for this extract to be further processed into a dry extract, which is subsequently mixed with or dissolved in a fat, wax or oil.

In a further preferred embodiment, in combination with any one of the embodiments listed above or below, for vaginal application, the composition according to the present invention may be administered via a vaginal ring. A vaginal ring is a flexible ring made of a polymeric material, which is inert in the human body and which is further coated with a composition of the present invention. The vaginal ring is inserted into the vagina and provides controlled release of drugs for intravaginal administration.

In another preferred embodiment, in combination with any one of the embodiments listed above or below, the composition is in the form of an aerosol or spray. Preferably a liquid or solid extract is used for this. In addition to the extract, the aerosol or spray can also contain pharmaceutically harmless substances, carrier media and auxiliary agents.

In a further preferred embodiment, in combination with any one of the embodiments listed above or below, the composition can also be administered as mouthwash or mouth spray.

The composition for use according to the invention is not restricted to people, but instead is also possible for animals, particularly mammals, such as pets or livestock.

The following examples explain the invention.

A composition comprising a concentrate or an extract of marrubium in combination with at least one concentrate or extract of another plant was tested with respect to its cell toxicity and cell viability, as well as its antiviral activity against HIV.

To determine the characteristics of the composition, the following examination methods were used.

Example

Preparation of an extract of *Marrubium vulgare*

The twigs and leaves are used for extraction. The plant material is dried at room temperature outdoors in the shade, down to a residual water content of a maximum of 10%. Subsequently, the plant parts are cut to a size of ≤ 8 mm.

The cut plant parts are submitted to percolation at 95 to 100° C with ten times the quantity of purified water Ph.Eur. for 4 to 5 hours. The solution produced is concentrated to 18 to 19% of the original volume by means of a plate evaporator at a steam temperature of 75 to 80 °C. The content of the dry substance amounts to approximately 45%.

Using an evaporator with agitator, the content of the dry substance is increased to 50 to 51% by means of heating the extract for four hours at 110 to 114 °C at a reduced pressure (0.6 bar).

Finally, vacuum belt drying at 16 mbar with descending temperature gradients (140 °C, 120 °C, 90 °C, 20 °C) is carried out. The content of the dry substance amounts to >92% with an overall yield of the dry extract of 22 to 25%. The extract is subsequently ground.

Preparation of an extract from *Ribes nigrum*

The twigs and leaves are used for extraction. The plant material is dried at room temperature outdoors in the shade, down to a residual water content of a maximum of 10%. Subsequently, the plant parts are cut to a size of ≤ 8 mm.

The cut plant parts are submitted to percolation at 95 to 100 °C with ten times the quantity of purified water Ph.Eur. for 4 to 5 hours. The solution produced is concentrated to 18 to 19% of the original volume by means of a plate evaporator at a steam temperature of 75 to 80 °C. The content of the dry substance amounts to approximately 45%.

Using an evaporator with agitator, the content of the dry substance is increased to 50 to 51% by means of heating the extract for four hours at 110 to 114 °C at a reduced pressure (0.6 bar).

Finally, vacuum belt drying at 16 mbar with descending temperature gradients (140 °C, 120 °C, 90 °C, 20 °C) is carried out. The content of the dry substance amounts to >92% with an overall yield of the dry extract of 22 to 25%. The extract is subsequently ground.

Preparation of an extract of *Cistus incanus*

The regrown aerial shoots (leaves, petals, and stems) were used for extraction. The plant material is dried outdoors in the shade at room temperature until it has a residual water content of no more than 10%. Subsequently, the plant parts are cut to a size of ≤ 8 mm.

The cut plant parts are subjected to a percolation at 95 °C with the tenfold amount of purified water Ph.Eur. for 4 to 5 hours. The solution obtained is concentrated at a steam temperature of 75 to 80 °C to 18 to 19% of the original volume by means of a plate evaporator. The content of dry substance is approximately 45%.

The content of dry substance is increased to 50 to 51% by means of a stirring evaporator by heating the extract for 4 hours at 110 to 114°C under reduced pressure (0.6 bar). Subsequently, the extract is boiled for 1 h at 100 °C to obtain a content dry substance of approximately 53%.

Finally, vacuum conveyor drying is performed at 16 mbar with decreasing temperature gradients (140 °C, 120 °C, 90 °C, 20 °C). The content of dry substance is 92 to 93%. The extract is subsequently ground.

Preparation of an extract of *Borago officinalis*

The twigs and leaves are used for extraction. The plant material is dried at room temperature outdoors in the shade, down to a residual water content of a maximum of 10%. Subsequently, the plant parts are cut to a size of ≤ 8 mm.

The cut plant parts are submitted to percolation at 95 to 100 °C with ten times the quantity of purified water Ph.Eur. for 4 to 5 hours. The solution produced is concentrated to 18 to 19% of the original volume by means of a plate evaporator at a steam temperature of 75 to 80 °C. The content of the dry substance amounts to approximately 45%.

Using an evaporator with agitator, the content of the dry substance is increased to 50 to 51% by means of heating the extract for four hours at 110 to 114 °C at a reduced pressure (0.6 bar).

Finally, vacuum belt drying at 16 mbar with descending temperature gradients (140 °C, 120 °C, 90 °C, 20 °C) is carried out. The content of the dry substance amounts to >92% with an overall yield of the dry extract of 22 to 25%. The extract is subsequently ground.

In vitro testing of substances of antiviral activity in the HeLa-P4 assay

Viruses: HIV-1 Lai (subtype B, X4); titer: 8 x $10^4$/ml; 20 µl/batch

Cells: HeLa-P4; 2.5 x $10^4$/batch

Principle of the assay

HeLa-P4 cells (from NIH AIDS Research and Reference Program) are a cell line, which was transfected with genes for the human CD4- and CCR5 receptor and which are therefore infectable with HIV-1 (the CXCR4-receptor is expressed on the cells). For quantification of the HIV infection, the cells carry a reporter gene, i.e. β-Galactosidase gen, which is under control of the HIV-1 promoter. After infection, the Tat-protein of HIV-1 transactivates the reporter gene and the degree of infection can be quantified by measurement of the enzyme activity in the lysate of the cells. An inhibition of HIV-1 infection results in a reduction of the β-Galactosidase activity with respect to the infection with viruses without substance addition. The background is defined by measuring the enzyme activity in cells, to which no virus was added. Since in principle a lower infection may also be based on toxic effects of the substances to the cells, in addition to said assay a cytotoxicity assay is also carried out.

Experimental

The test substances are prepared by adding 1 mg of each extract/concentrate in an Eppendorf vial and dissolving the sample in 1 ml of RPMI medium. The solution is filtered under sterile conditions and placed in a new Eppendorf vial. As a positive control, the T20-peptide (a known HIV-1 entry inhibitor; Enfuvirtide) is used. Dilution series of the test compounds alone, combinations thereof as well as of T20 in cell culture medium were prepared, wherein the samples have concentrations of 5, 10, 25, and 50 µg/ml for *Marrubium vulgare*, *Cistus incanus* and *Ribes nigrum* and combinations of extracts of *Marrubium vulgare* and *Cistus incanus*, and *Marrubium vulgare* and *Ribes nigrum.* For *Marrubium vulgare*, *Borago officinalis* and combinations of extracts of *Marrubium vulgare* and *Borago officinalis* concentrations of 5 and 50 µg/ml were tested. The given concentrations in the combinations relate to the concentration of each single extract, e.g. a concentration of 5 µg/ml means 5 µg/ml of *Marrubium vulgare* and 5 µg/ml of the extract of the other plant (*Cistus incanus*, *Ribes nigrum, Borago officinalis*).
The positive control T20 is used in concentrations of 100 and 200 nM. Each of the substance concentrations was tested in triplicates.
Day 1: Each of the substance dilutions of *Marrubium vulgare* (5, 10, 25, 50, and 125 µg/ml), *Ribes nigrum* (5, 10, 25, and 50 µg/ml), *Cistus incanus* (5, 10, 25, and 50 µg/ml) and *Borago officinalis* (5 and 50 µg/m) is mixed with 20 µl of the HIV-1 Lai virus stock (titer 8x$10^4$/ml), vortexed, and pre-incubated at 37 °C for 30 minutes. The virus/substance mixture is added to 2.5 x $10^4$ HeLa-P4 cells corresponding to an *m.o.i.* of 0.06, vortexed and incubated for 2 h at 37 °C. Afterwards the supernatant is removed, the cells are washed with medium and 100 µl of fresh medium is added. The cells are incubated for 2 days at 37 °C.
Accordingly, each of the substance dilutions of combinations of *Marrubium vulgare* and *Ribes nigrum* (5, 10, 25, and 50 µg/ml), *Marrubium vulgare* and *Cistus incanus* (5, 10, 25, and 50 µg/ml) and *Marrubium vulgare* and *Borago officinalis* (5 and 50 µg/ml) is mixed with 20 µl of the HIV-1 Lai virus stock (titer 8x$10^4$/ml), vortexed, and pre-incubated at 37 °C for 30 minutes. The virus/substance mixture is added to 2.5 x $10^4$ HeLa-P4 cells, vortexed and incubated for 2 h at 37 °C. Afterwards the supernatant is removed, the cells are washed with medium and 100 µl of fresh medium is added. The cells are incubated for 2 days at 37 °C.
Day 3: The supernatant is removed, the cells are washed with 100 µl of medium and lysed in 50 µl of lysis buffer per each well (2.5 ml glycerol; 1.25 ml MES-Tris, 25 µl 1 M DTT, 250 µl Triton X100, $H_2O$ ad 25 ml) for 10 minutes on ice and removed at -80 °C. In a white micro titer plate, 34 µl of reaction buffer (15 µl 1 M $MgCl_2$, 3 ml 0.5 M $NaH_2PO_4$/$Na_2HPO_4$, 150 µl Galacton 100x, ad 15 ml $H_2O$) is placed in each well and 20 µl of cell lysate is added. The micro titer plate is shaken for 45 to 60 minutes in the dark and subsequently after addition of 25 µl of amplifier (80 µl 10 M NaOH, 400 µl 10x Emerald (Applied Biosystems), ad 4 ml $H_2O$) per each well, the enzyme activity is measured on a luminometer (Lumistar Galaxy, BMG Labtechnologies, Offenburg; settings: microplate: Dynatech 96, number of intervals: 50, measurement interval time: 0.2 seconds, positioning delay: 0.5 seconds, total measurement time/well: 10 seconds, gain: 250, start interval: 1, stop interval: 50).
The following results were obtained:

Table 1: Antiviral activity of an extract of *Ribes nigrum* against HIV-1

| | LU (luminescence units) | | | |
|---|---|---|---|---|
| | Trial 1 | Trial 2 | Trial 3 | Average |
| 50 µg/ml | 3309 | 9225 | 14648 | 9061 |
| 25 µg/ml | 23301 | 29352 | 28750 | 27134 |
| 10 µg/ml | 38630 | 46017 | 45666 | 43438 |
| 5 µg/ml | 40546 | 44697 | 36343 | 40529 |

Table 2: Antiviral activity of an extract of *Marrubium vulgare* against HIV-1

| | LU (luminescence units) | | | |
|---|---|---|---|---|
| | Trial 1 | Trial 2 | Trial 3 | Average |
| 50 µg/ml | 23812 | 19667 | 22527 | 22002 |
| 25 µg/ml | 38795 | 33459 | 35610 | 35955 |
| 10 µg/ml | 46347 | 41134 | 49508 | 45663 |
| 5 µg/ml | 47572 | 59258 | 53881 | 53570 |

Table 3: Antiviral activity of an extract of *Marrubium vulgare* and *Ribes nigrum* against HIV-1

| | LU (luminescence units) | | | |
|---|---|---|---|---|
| | Trial 1 | Trial 2 | Trial 3 | Average |
| 50 µg/ml | 5859 | 5549 | 6327 | 5912 |
| 25 µg/ml | 17844 | 18416 | 17081 | 17780 |
| 10 µg/ml | | 32924 | 28383 | 30654 |
| 5 µg/ml | | 41709 | 39181 | 40445 |

Table 4: Antiviral activity of an extract of *Cistus incanus* against HIV-1

| | LU (luminescence units) | | | |
|---|---|---|---|---|
| | Trial 1 | Trial 2 | Trial 3 | Average |
| 50 µg/ml | 5744 | 4968 | 7465 | 6059 |
| 25 µg/ml | 13197 | 15337 | 15631 | 14722 |
| 10 µg/ml | 35454 | 35572 | 26946 | 32657 |
| 5 µg/ml | 47169 | 49408 | 44290 | 46956 |

Table 5: Antiviral activity of an extract of *Marrubium vulgare* and *Cistus incanus* against HIV-1

| | LU (luminescence units) | | | |
|---|---|---|---|---|
| | Trial 1 | Trial 2 | Trial 3 | Average |
| 50 µg/ml | 3241 | 3859 | 4967 | 4022 |
| 25 µg/ml | 3654 | 6676 | 2750 | 4360 |
| 10 µg/ml | | 12914 | 23333 | 18124 |

(continued)

| | LU (luminescence units) | | | |
|---|---|---|---|---|
| | Trial 1 | Trial 2 | Trial 3 | Average |
| 5 µg/ml | 24563 | 31623 | 28075 | 28087 |

Table 6: Antiviral activity of an extract of *Borago officinalis* against HIV-1

| | LU (luminescence units) | | | |
|---|---|---|---|---|
| | Trial 1 | Trial 2 | Trial 3 | Average |
| 50 µg/ml | 671 | 614 | 594 | 626 |
| 5 µg/ml | 20018 | 21337 | 21564 | 20973 |

Table 7: Antiviral activity of an extract of *Marrubium vulgare* against HIV-1

| | LU (luminescence units) | | | |
|---|---|---|---|---|
| | Trial 1 | Trial 2 | Trial 3 | Average |
| 50 µg/ml | 9690 | 11281 | 14882 | 11951 |
| 5 µg/ml | 31560 | 34875 | 32741 | 33059 |

Table 8: Antiviral activity of an extract of *Marrubium vulgare* and *Borago officinalis* against HIV-1

| | LU (luminescence units) | | | |
|---|---|---|---|---|
| | Trial 1 | Trial 2 | Trial 3 | Average |
| 50 µg/ml | 603 | 629 | 614 | 615 |
| 5 µg/ml | 18922 | 16873 | 16487 | 17427 |

Table 8: Antiviral activity of T20 against HIV-1

| | LU (luminescence units) | | | |
|---|---|---|---|---|
| | Trial 1 | Trial 2 | Trial 3 | Average |
| 200 nM | 9407 | 7531 | 9468 | 8802 |
| 100 nM | 23855 | 13888 | 12937 | 16893 |

**Cytotoxicity test**

Principle of the assay

The cytotoxicity assay analyses substances in view of toxic effects to the cells.

HeLa-P4 cells were used for the cytotoxicity assay. A commercially avaiable kit was used (ViaLight® Plus Kit, Lonza, Rockland, USA). The principle of the kit is based on measurements of the amount of ATP in the cytoplasma of metabolic active, i. e. living, cells. Any disturbance in the viability of cells results in reduction of the amount of ATP. The amount of ATP is determined by bioluminometric measurements, wherein light is formed by the enzyme luciferase from ATP and the substrate luciferin in the presence of oxygen. The intensity of emmitted light is proportional to the ATP concentration and is determined on a luminometer (Lumistar Galaxy, BMG Labtechnologies, Offenburg).

<u>Experimental</u>

The test substances are prepared as described above in view of the in vitro testing of antiviral activity in the HeLa-P4 assay. Each of the substance concentrations was tested in triplicates.

Day 1: Plating of 2.5 x $10^4$ HeLa-P4 cells in 100 $\mu$l of medium (DMEM, 10% FCS, 2% L-glutamine, 1% penicillin/streptomycin) per each well of a 96-well microtiter plate, incubation by 37 °C overnight.

Day 2: The medium is removed and the cells are washed with 100 $\mu$l of PBS. 100 $\mu$l of each of the substance dilutions (1.25, 2.5, 5, 10, 25, 50 and 125 $\mu$g/ml) were placed in each well of a 96-well microtiter plate and incubated for 2 h at 37 °C, the T20 dilutions (200 nM and 100 nM) are treated in the same manner and the cells are incubated for 2 days at 37 °C.

Day 4: The cells were removed from the incubator. The supernatant is removed, the cells are washed three times with 100 $\mu$l of PBS per each well and lysed in 50 $\mu$l of lysis buffer (2.5 ml glycerol; 1.25 ml MES-Tris, 25 $\mu$l 1 M DTT, 250 $\mu$l Triton X100, $H_2O$ ad 25 ml). The plate was left for 10 minutes at room temperature.

In a white micro titer plate, 100 $\mu$l of AMR-Plus reagent were placed in each well and 35 $\mu$l of the cell lysate is added (without air bubbles). The micro titer plate is incubated for 2 min at room temperature and then measured on a luminometer (Lumistar Galaxy, BMG Labtechnologies, Offenburg; settings: microplate: Dynatech 96, number of intervals: 40, measurement interval time: 0.25 seconds, positioning delay: 0.5 seconds, total measurement time/well: 10 seconds, gain: 190, start interval: 1, stop interval: 40, test type: well mode, reading direction: horizontal).

Table 9: Results of cytotoxicity test of an extract of *Marrubium vulgare* and *Borago officinalis*

| | LU (luminescence units) | | |
|---|---|---|---|
| | Trial 1 | Trial 2 | Average |
| 125 $\mu$g/ml | 325905 | 297969 | 311937 |
| 50 $\mu$g/ml | 327401 | 341117 | 334259 |
| 25 $\mu$g/ml | 303400 | 311049 | 307225 |
| 5 $\mu$g/ml | 301295 | 313709 | 307502 |
| 2.5 $\mu$g/ml | 292554 | 286802 | 289678 |

Table 10: Results of cytotoxicity test of an extract of *Ribes nigrum*

| | LU (luminescence units) | | | |
|---|---|---|---|---|
| | Trial 1 | Trial 2 | Trial 3 | Average |
| 50 $\mu$g/ml | 115959.7 | 124045.9 | 124979.9 | 121662 |
| 25 $\mu$g/ml | 124699.9 | 124859.2 | 112629.0 | 120729 |
| 10 $\mu$g/ml | 112048.8 | 122400.7 | 124980.9 | 119810 |
| 5 $\mu$g/ml | 117762.2 | 104548.5 | 124804.8 | 115705 |
| 2.5 $\mu$g/ml | 117610.7 | 120772.2 | 109540.3 | 115974 |
| 1.25 $\mu$g/ml | 122335.0 | 124786.7 | 123295.6 | 123472 |

Table 11: Results of cytotoxicity test of *Marrubium vulgare*

| | LU (luminescence units) | | | |
|---|---|---|---|---|
| | Trial 1 | Trial 2 | Trial 3 | Average |
| 50 $\mu$g/ml | 115049.7 | 89988.9 | 124359.1 | 109799 |
| 25 $\mu$g/ml | 122153.1 | 113232.2 | 97501.2 | 110962 |
| 10 $\mu$g/ml | 112908.8 | 124445.9 | 121528.8 | 119628 |
| 5 $\mu$g/ml | 114670.7 | 108611.7 | 121951.8 | 115078 |

(continued)

|  | LU (luminescence units) | | | |
|---|---|---|---|---|
|  | Trial 1 | Trial 2 | Trial 3 | Average |
| 2.5 μg/ml | 121029.1 | 117405.9 | 103446.7 | 113961 |
| 1.25 μg/ml | 118215.3 | 124221.6 | 122505.1 | 121647 |

Table 12: Results of cytotoxicity test of *Cistus incanus*

|  | LU (luminescence units) | | | |
|---|---|---|---|---|
|  | Trial 1 | Trial 2 | Trial 3 | Average |
| 50 μg/ml | 119952.4 | 117792.1 | 118907.6 | 118884 |
| 25 μg/ml | 124981.3 | 124923.9 | 113955.2 | 121287 |
| 10 μg/ml | 124976.3 | 124123.7 | 124964.4 | 124688 |
| 5 μg/ml | 101084.0 | 111526.3 | 120326.3 | 110979 |
| 2.5 μg/ml | 124906.4 | 124062.3 | 109011.7 | 119327 |
| 1.25 μg/ml | 122223.9 | 124977.7 | 124981.0 | 124061 |

Table 13: Results of cytotoxicity test of *Marrubium vulgare* and *Cistus incanus*

|  | LU (luminescence units) | | | |
|---|---|---|---|---|
|  | Trial 1 | Trial 2 | Trial 3 | Average |
| 50 μg/ml | 113693.6 | 110816.6 | 107174.8 | 110562 |
| 25 μg/ml | 96725.0 | 108645.0 | 115214.3 | 106861 |
| 10 μg/ml | 93303.0 | 105291.5 | 108701.3 | 102432 |
| 5 μg/ml | 101958.7 | 105165.8 | 80300.4 | 95808 |
| 2.5 μg/ml | 103557.5 | 109785.6 | 100797.7 | 104714 |
| 1.25 μg/ml | 85372.6 | 105513.8 | 93986.0 | 94957 |

Table 14: Results of cytotoxicity test of *Marrubium vulgare* and *Ribes nigrum*

|  | LU (luminescence units) | | | |
|---|---|---|---|---|
|  | Trial 1 | Trial 2 | Trial 3 | Average |
| 50 μg/ml | 107334.0 | 116568.1 | 101894.7 | 108599 |
| 25 μg/ml | 107678.2 | 112851.4 | 114504.7 | 111678 |
| 10 μg/ml | 92110.9 | 114361.9 | 115605.1 | 107359 |
| 5 μg/ml | 101998.0 | 109777.8 | 108072.8 | 106616 |
| 2.5 μg/ml | 96891.9 | 113231.4 | 109932.7 | 106685 |
| 1.25 μg/ml | 85298,1 | 107262.6 | 108903.1 | 100488 |

Table 15: Results of cytotoxicity test without drug

| LU (luminescence units) | | | |
|---|---|---|---|
| Trial 1 | Trial 2 | Trial 3 | Average |
| 88125.6 | 83887.9 | 84299.3 | 95960.7 |

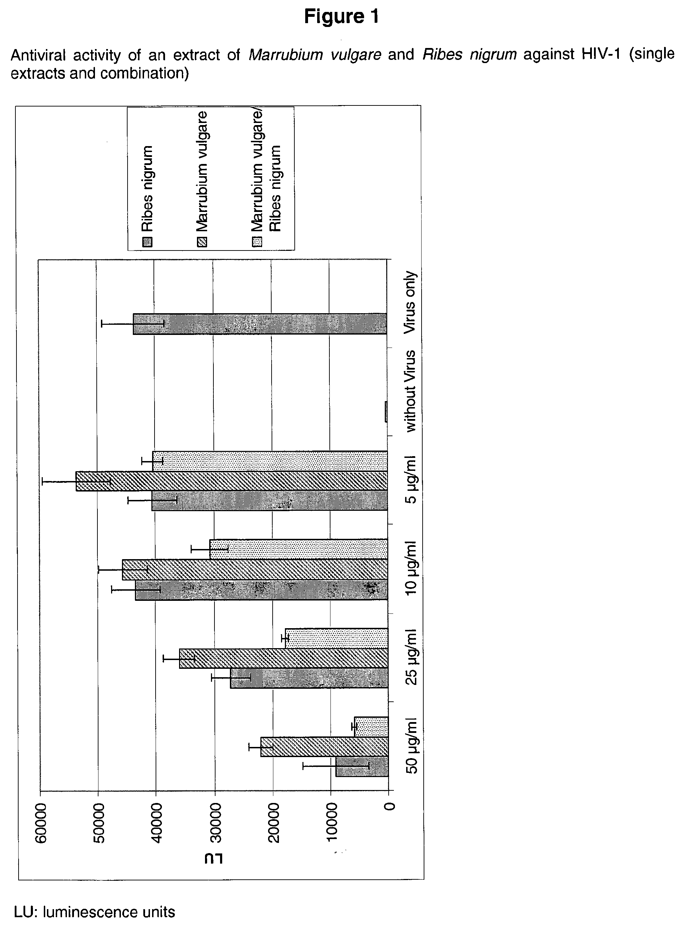

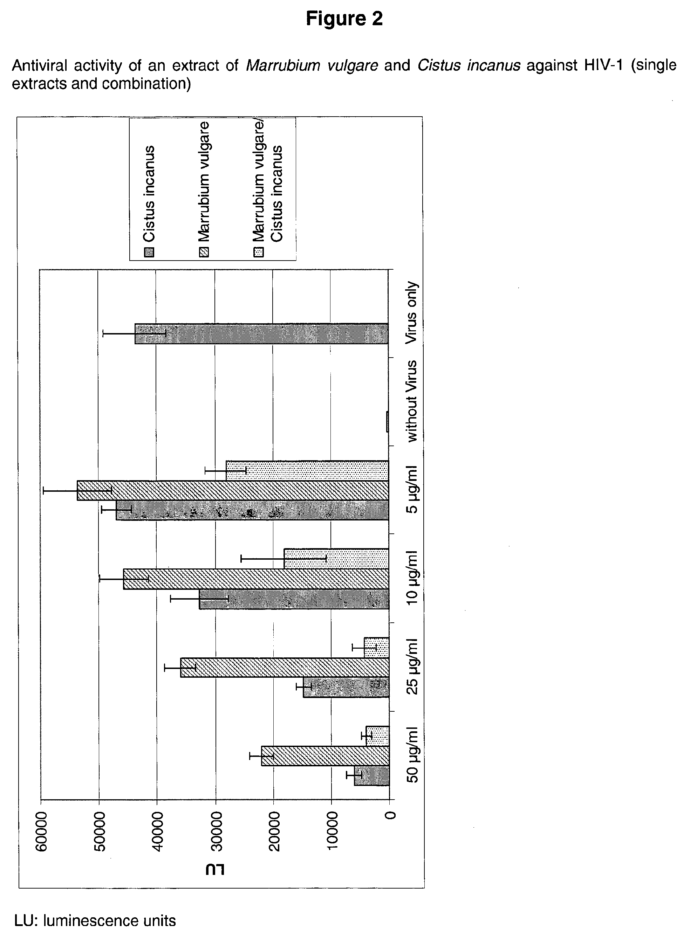

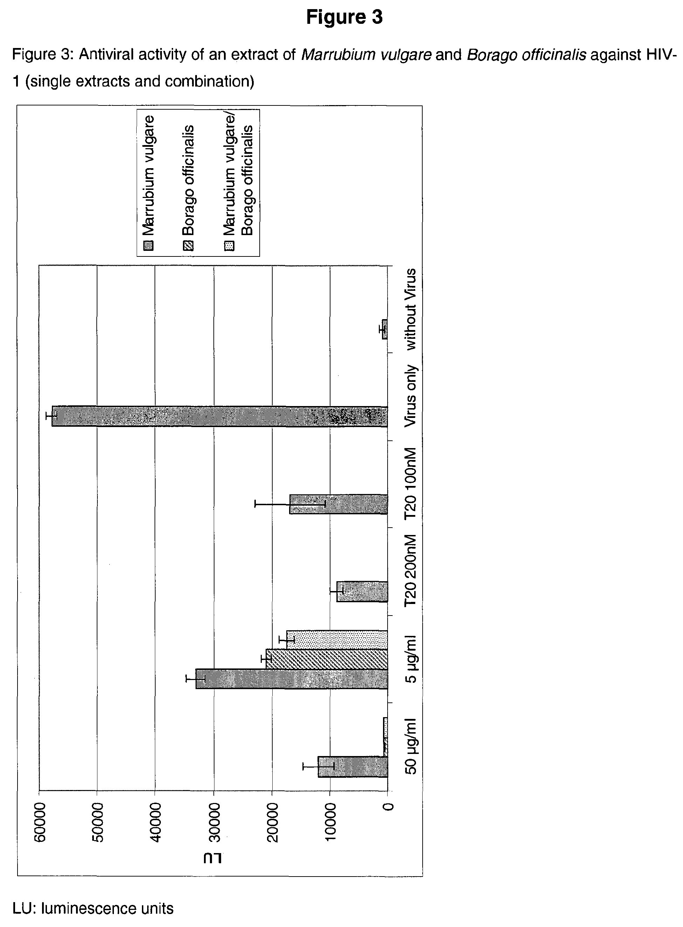

In the result, as can be seen from Figures 1 to 3, the composition comprising a concentrate or an extract of marrubium in combination with at least one concentrate or extract of another plant was able to inhibit the infection caused by HIV. It can be furthermore seen from the results that a better inhibition is obtained when using the combinations of a concentrate or an extract of marrubium with a concentrate or an extract of another plant compared to a single plant extract.

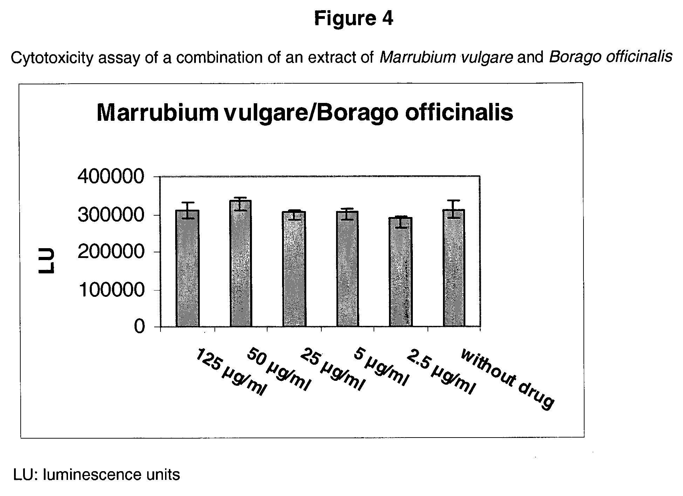

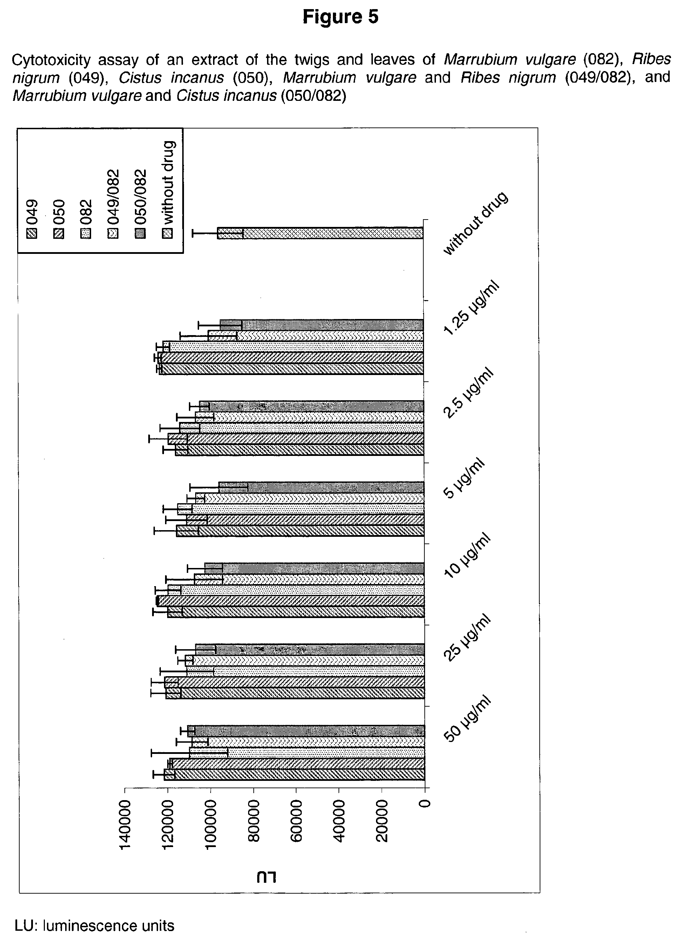

As can be seen from Figures 4 and 5, in the concentration used, the composition had no detectable damaging influences on the cells.

**Determination of synergistic effects (chessboard titration)**

Cells: PM-1; $2 \times 10^4$ /batch

Pseudovirus: Lenti HXB2env GFP; titer: $6 \times 10^4$/batch (m. o. i. = 3)

Medium: RPMI, 10% FCS, 4mM L-Glutamine

Test substances: *Ribes nigrum*, *Cistus incanus*, *Marrubium vulgare*

End concentration of substances:

*Ribes nigrum / Marrubium vulgare*:

*Ribes nigrum:* 50; 25; 12.5; 6.25; 3.1; 1.5 $\mu$g /ml

*Marrubium vulgare*: 100; 50; 25; 12.5; 6.25; 3.1 $\mu$g /ml

*Cistus incanus / Marrubium vulgare*:

*Cistus incanus*: 6.25; 3.1; 1.5; 0.75; 0.375; 0.187 $\mu$g /ml

*Marrubium vulgare*: 100; 50; 25; 12.5; 6.25; 3.1 $\mu$g /ml

Principle of the assay

In this assay, the antiviral activity of the combinations of substances, which alone already have an antiviral activity against retroviruses, was examined. HIV-1 Pseudoviruses Lenti HXB2env GFP, having GFP (green fluorescent protein) as marker gen were used. The infectivity of cells is determined by FACS (fluorescent activated cell sorting) as number of green fluorescing cells.

Experimental

Day 1: Of each of the test substances samples having a concentration twofold of those indicated above were prepared for the respective combinations. The virus was added in a concentration of $6 \times 10^4$/batch (m.o.i. = 3). Dilution series were prepared by adding medium wherein the virus is present at $6 \times 10^4$/batch to obtain the test substances in the above given end concentrations. In a 96-well plate 50 $\mu$l of the substance dilutions were combined pairwise, such that the concentration of the extracts decreases from left to right and top to bottom. In separate plates 50 μl of each of the test substance dilutions is mixed with 50 μl of medium, wherein the virus is present in the given concentration, in order to compare the antiviral effect of the substance combinations to the antiviral activity of the single substances. Furthermore, in two separate wells 100 μl of medium with virus is plated in order to determine the virus-only value. The plates were incubated for 30 min at 37 °C, afterwards 3 μl of cell suspension containing 2 x 10⁴ cells were added to each well and incubated for 24 h at 37 °C.

Day 2: To each of the wells 100 μl of fresh medium was added and incubation was continued at 37 °C.

Day 5: Cells were resuspended in their medium, removed from the wells and transfered into round bottom FACS vials. The percentages of GFP positive cells were measured directly on a FACS-Calibur to determine the number cells transduced with the HXB2env pseudovirus. The percentage of GFP positive cells was determined using the software Cellquest Pro in comparison to the number of GFP positive cells in the "virus only" sample. This allows to determine the inhibiton by the different substance combinations.

Table 16: Results of chessboard titration of *Marrubium vulgare* (082) and *Ribes nigrum* (049)

| 082<br>049 | 100 μg/mL | 50 μg/mL | 25 μg/mL | 12.5 μg/mL | 6.25 μg/mL | 3.1 μg/mL |
|---|---|---|---|---|---|---|
| 50 μg/mL | 1.51%<br>049=5.02%<br>082=6.32% | 2.8%<br>049=5.02%<br>082=11.8% | 3.85%<br>049=5.02%<br>082=16.2% | 3.8%<br>049=5.02%<br>082=14.54% | 4.4%<br>049=5.02%<br>082=23.95% | 4.55%<br>049=5.02%<br>082=25.12% |
| 25 μg/mL | 3.3%<br>049=9.05%<br>082=6.32% | 5.3%<br>049=9.05%<br>082=11.8% | 6.3%<br>049=9.05%<br>082=16.2% | 7.5%<br>049=9.05%<br>082=14.54% | 7.95%<br>049=9.05%<br>082=23.95% | 7.6%<br>049=9.05%<br>082=25.12% |
| 12.5 μg/mL | 3.9%<br>049=13.75%<br>082=6.32% | 6.7%<br>049=13.75%<br>082=11.8% | 7.95%<br>049=13.75%<br>082=16.2% | 9.1%<br>049=13.75%<br>082=14.54% | 10.7%<br>049=13.75%<br>082=23.95% | 11.4%<br>049=13.75%<br>082=25.12% |
| 6.25 μg/mL | 4.55%<br>049=16.8%<br>082=6.32% | 8.8%<br>049=16.8%<br>082=11.8% | 11%<br>049=16.8%<br>082=16.2% | 12.7%<br>049=16.8%<br>082=14.54% | 13.15%<br>049=16.8%<br>082=23.95% | 14.65%<br>049=16.8%<br>082=25.12% |
| 3.1 μg/mL | 5.6%<br>049=21.9%<br>082=6.32% | 8.8%<br>049=21.9%<br>082=11.8% | 12.1%<br>049=21.9%<br>082=16.2% | 16.55%<br>049=21.9%<br>082=14.54% | 15.9%<br>049=21.9%<br>082=23.95% | 15.5%<br>049=21.9%<br>082=25.12% |
| 1.5 μg/mL | 5.8%<br>049=21.2%<br>082=6.32% | 11.85%<br>049=21.2%<br>082=11.8% | 14.4%<br>049=21.2%<br>082=16.2% | 18.15%<br>049=21.2%<br>082=14.54% | 19.25%<br>049=21.2%<br>082=23.95% | 20.35%<br>049=21.2%<br>082=25.12% |

The first column of Table 16 describes the concentration of *Ribes nigrum,* the first line the concentration of *Marrubium vulgare*. The first value in each table cell indicates the percentage of infected cells after the treatment with the substance combinations of *Marrubium vulgare* and *Ribes nigrum,* the second value indicates the percentage ofinfected cells for a treatment with *Ribes nigrum* alone and the third value the percentage of infected cells for a treatment with *Marrubium vulgare* alone.

The virus-only value in this experiment was 29.5%.

Table 17: Results of chessboard titration of *Marrubium vulgare* (082) and *Cistus incanus* (050)

| 050<br>082 | 6.25 μg/mL | 3.1 μg/mL | 1.5 μg/mL | 0.75 μg/mL | 0.375 μg/mL | 0.187 μg/mL |
|---|---|---|---|---|---|---|
| 100 μg/mL | 1.47%<br>082=3.7%<br>050=6.86% | 1.92%<br>082=3.7%<br>050=9.15% | 2.78%<br>082=3.7%<br>050=14.06% | 2.73%<br>082=3.7%<br>050=17.8% | 2.98%<br>082=3.7%<br>050=19.13% | 3.36%<br>082=3.7%<br>050=23% |
| 50 μg/mL | 3.46%<br>082=7.2%<br>050=6.86% | 4%<br>082=7.2%<br>050=9.15% | 4.03%<br>082=7.2%<br>050=14.06% | 4.9%<br>082=7.2%<br>050=17.8% | 3.63%<br>082=7.2%<br>050=19.13% | 3.6%<br>082=7.2%<br>050=23% |

(continued)

| 050 082 | 6.25 µg/mL | 3.1 µg/mL | 1.5 µg/mL | 0.75 µg/mL | 0.375 µg/mL | 0.187 µg/mL |
|---|---|---|---|---|---|---|
| 25 µg/mL | 4.59% 082=10.43% 050=6.86% | 4.61% 082=10.43% 050=9.15% | 6.4% 082=10.43% 050=14.06% | 5.59% 082=10.43% 050=17.8% | 4.46% 082=10.43% 050=19.13% | 5.4% 082=10.43% 050=23% |
| 12.5 µg/mL | 4.72% 082=13.33% 050=6.86% | 5.6% 082=13.33% 050=9.15% | 7% 082=13.33% 050=14.06% | 7.38% 082=13.33% 050=17.8% | 5.38% 082=13.33% 050=19.13% | 6.95% 082=13.33% 050=23% |
| 6.25 µg/mL | 4.2% 082=15.48% 050=6.86% | 5.54% 082=15.48% 050=9.15% | 8.76% 082=15.48% 050=14.06% | 9.25% 082=15.48% 050=17.8% | 9.17% 082=15.48% 050=19.13% | 9.35% 082=15.48% 050=23% |
| 3.1 µgmL | 5.7% 082=19.2% 050=6.86% | 7.52% 082=19.2% 050=9.15% | 10.56% 082=19.2% 050=14.06% | 13.3% 082=19.2% 050=17.8% | 13.29% 082=19.2% 050=19.13% | 14.45% 082=19.2% 050=23% |

The first column of Table 17 describes the concentration of *Marrubium vulgare*, the first line the concentration of *Cistus incanus*. The first value in each table cell indicates the percentage of infected cells after the treatment with the substance combinations of *Marrubium vulgare* and *Cistus incanus*, the second value indicates the percentage of infected cells for a treatment with *Marrubium vulgare* alone and the third value the percentage of infected cells for a treatment with *Cistus incanus* alone.

The virus-only value in this experiment was 22.6%.

Determination of $IC_{50}$ and $IC_{75}$ values of single substances

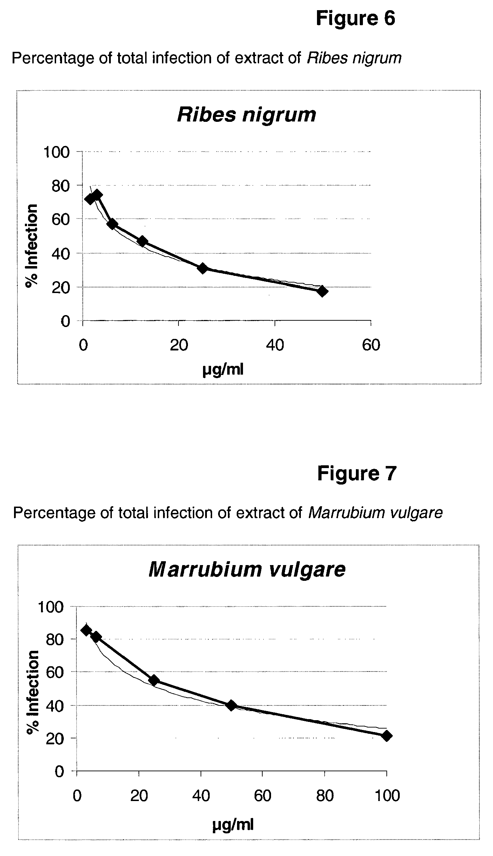

Table 18: Infected cells for single substances *Marrubium vulgare* and *Ribes nigrum* (Figures 6 and 7)

| concentration (µg/mL) | *Ribes nigrum* infected cells (%) | Percent of total infection (%) | *Marrubium vulgare* infected cells (%) | Percent of total infection (%) |
|---|---|---|---|---|
| 1.5 | 21.2 | 71.86 | | |
| 3.1 | 21.9 | 74.24 | 25.12 | 85.15 |
| 6.25 | 16.8 | 56.95 | 23.95 | 81.19 |
| 12.5 | 13.75 | 46.61 | | |
| 25 | 9.05 | 30.68 | 16.2 | 54.92 |
| 50 | 5.02 | 17.02 | 11.8 | 40.00 |
| 100 | | | 6.32 | 21.42 |

The second and fourth columns of Table 18 show the percentages of infected cells and were taken from Table 16 at the respective concentrations. In the third and fifth column the percentages of infected cells based on the virus-only value (29.5%) are given, i.e. 29.5% corresponds to 100% of total infection. The respective graphs of said values are shown in Figures 6 and 7.

The following $IC_{50}$ and $IC_{75}$ values were obtained from the graphs. The $IC_{50}$ value is the half maximal inhibitory concentration and indicates how much of a particular drug or other substance is needed to inhibit a given biological process by half. Accordingly, the $IC_{75}$ value indicates how much of a particular drug or other substance is needed to inhibit a given biological process by 75%. Thus, the $IC_{50}$ value corresponds to the X-axis value at 50% of infection, the $IC_{75}$ value corresponds to the X-axis value at 25% of infection.

*Ribes nigrum:* $IC_{50}$ = 8.5 µg/ml, $IC_{75}$ = 37.3 µg/ml

*Marrubium vulgare*: $IC_{50}$ = 26.8 $\mu$g/ml, $IC_{75}$ =103.2 $\mu$g/ml

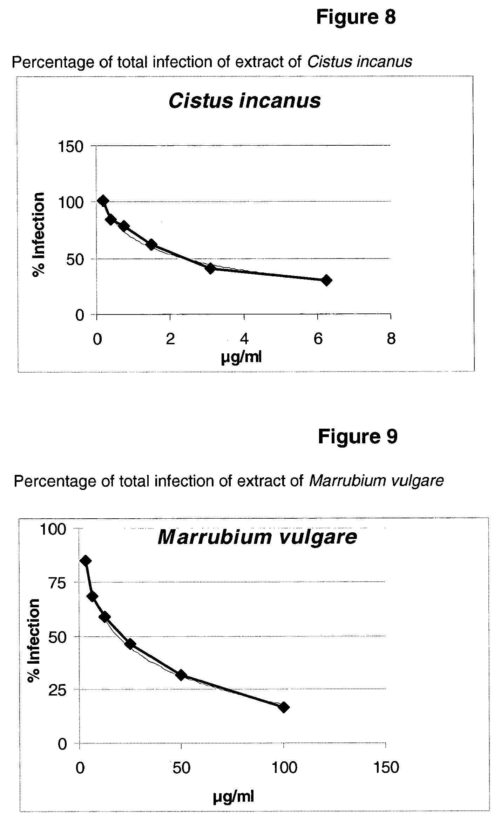

Table 19: Infected cells for single substance *Cistus incanus* (Figure 8)

| *Cistus incanus* concentration ($\mu$g/mL) | *Cistus incanus* infected cells (%) | Percent of total infection (%) |
|---|---|---|
| 0.187 | 23 | 101.77 |
| 0.375 | 19.13 | 84.65 |
| 0.75 | 17.8 | 78.76 |
| 1.5 | 14.06 | 62.21 |
| 3.1 | 9.15 | 40.49 |
| 6.25 | 6.86 | 30.35 |

Table 20: Infected cells for single substance *Marrubium vulgare* (Figure 9)

| *Marrubium vulgare* concentration ($\mu$g/mL) | *Marrubium vulgare* infected cells (%) | Percent of total infection (%) |
|---|---|---|
| 3.1 | 19.2 | 84.96 |
| 6.25 | 15.48 | 68.50 |
| 12.5 | 13.33 | 58.98 |
| 25.0 | 10.43 | 46.15 |
| 50.0 | 7.2 | 31.86 |
| 100.0 | 3.7 | 16.37 |

The second column of Tables 19 and 20 show the percentages of infected cells and were taken from Table 17 at the respective concentrations. In the third column the percentages of infected cells based on the virus-only value (22.6 %) are given, i.e. 22.6 % corresponds to 100 % of total infection. The respective graphs of said values are shown in Figures 8 and 9.

The following $IC_{50}$ and $IC_{75}$ values were obtained from the graphs.

*Cistus incanus*: $IC_{50}$ = 2.37 $\mu$g/ml, $IC_{75}$ = 7.98 $\mu$g/ml

*Marrubium vulgare*: $IC_{50}$ = 18.7 $\mu$g/ml, $IC_{75}$ = 62.8 $\mu$g/ml

Determination of $IC_{50}$ and $IC_{75}$ values of combinations

In the same manner as indicated above for the single substances, the percentages of total infection for combinations of *Marrubium vulgare* and *Ribes nigrum* were determined. The virus-only value of 29.5% corresponds to 100% of total infection.

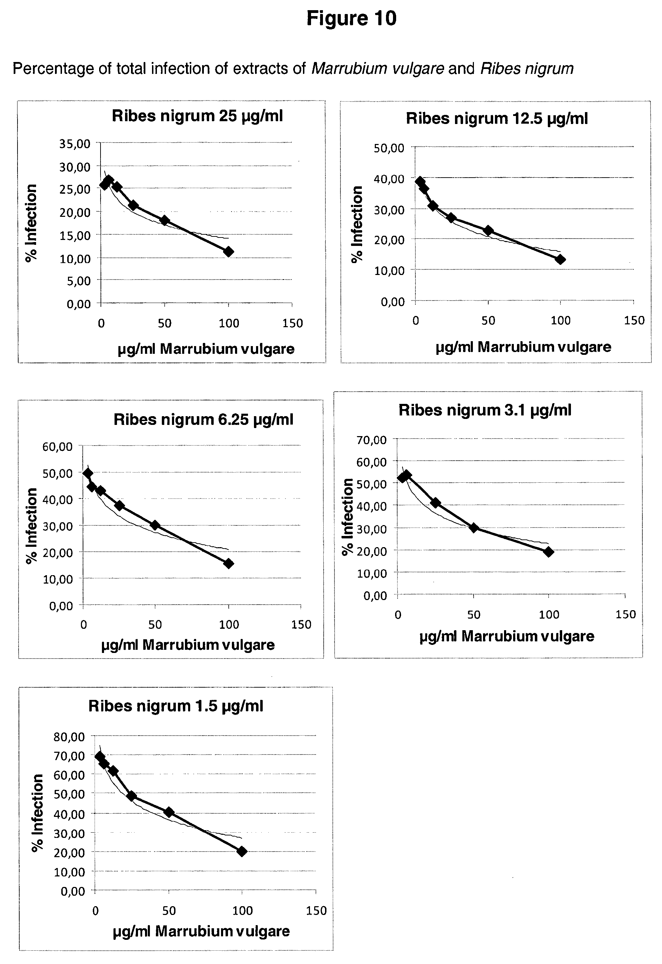

Table 21: Infected cells for combination of *Marrubium vulgare* and *Ribes nigrum* (Figure 10)

| *Marrubium vulgare* concentration ($\mu$g/mL) | Infected cells in combination with 50 $\mu$g/ml of *Ribes nigrum* (%) | Percent of total infection (%) | Infected cells in combination with 25 $\mu$g/ml of *Ribes nigrum* (%) | Percent of total infection (%) | Infected cells in combination with 12.5 $\mu$g/ml of *Ribes nigrum* (%) | Percent of total infection (%) |
|---|---|---|---|---|---|---|
| 3.1 | 4.55 | 15.42 | 7.6 | 25.76 | 11.4 | 38.64 |
| 6.25 | 4.40 | 14.92 | 7.95 | 26.95 | 10.7 | 36.27 |
| 12.5 | 3.80 | 12.88 | 7.5 | 25.42 | 9.1 | 30.85 |

(continued)

| *Marrubium vulgare* concentration (μg/mL) | Infected cells in combination with 50 μg/ml of *Ribes nigrum* (%) | Percent of total infection (%) | Infected cells in combination with 25 μg/ml of *Ribes nigrum* (%) | Percent of total infection (%) | Infected cells in combination with 12.5 μg/ml of *Ribes nigrum* (%) | Percent of total infection (%) |
|---|---|---|---|---|---|---|
| 25 | 3.85 | 13.05 | 6.3 | 21.36 | 7.95 | 26.95 |
| 50 | 2.80 | 9.49 | 5.3 | 17.97 | 6.7 | 22.71 |
| 100 | 1.51 | 5.12 | 3.3 | 11.19 | 3.9 | 13.22 |

Table 22: Infected cells for combination of *Marrubium vulgare* and *Ribes nigrum* (Figure 10)

| *Marrubium vulgare* concentration (μg/mL) | Infected cells in combination with 6.25 μg/ml of *Ribes nigrum* (%) | Percent of total infection (%) | Infected cells in combination with 3.1 μg/ml of *Ribes nigrum* (%) | Percent of total infection (%) | Infected cells in combination with 1.5 μg/ml of *Ribes nigrum* (%) | Percent of total infection (%) |
|---|---|---|---|---|---|---|
| 3.1 | 14.65 | 49.66 | 15.50 | 52.54 | 20.35 | 68.98 |
| 6.25 | 13.15 | 44.58 | 15.90 | 53.90 | 19.25 | 65.25 |
| 12.5 | 12.70 | 43.05 | | | 18.15 | 61.53 |
| 25 | 11.00 | 37.29 | 12.10 | 41.02 | 14.40 | 48.81 |
| 50 | 8.80 | 29.83 | 8.80 | 29.83 | 11.85 | 40.17 |
| 100 | 4.55 | 15.42 | 5.60 | 18.98 | 5.80 | 19.66 |

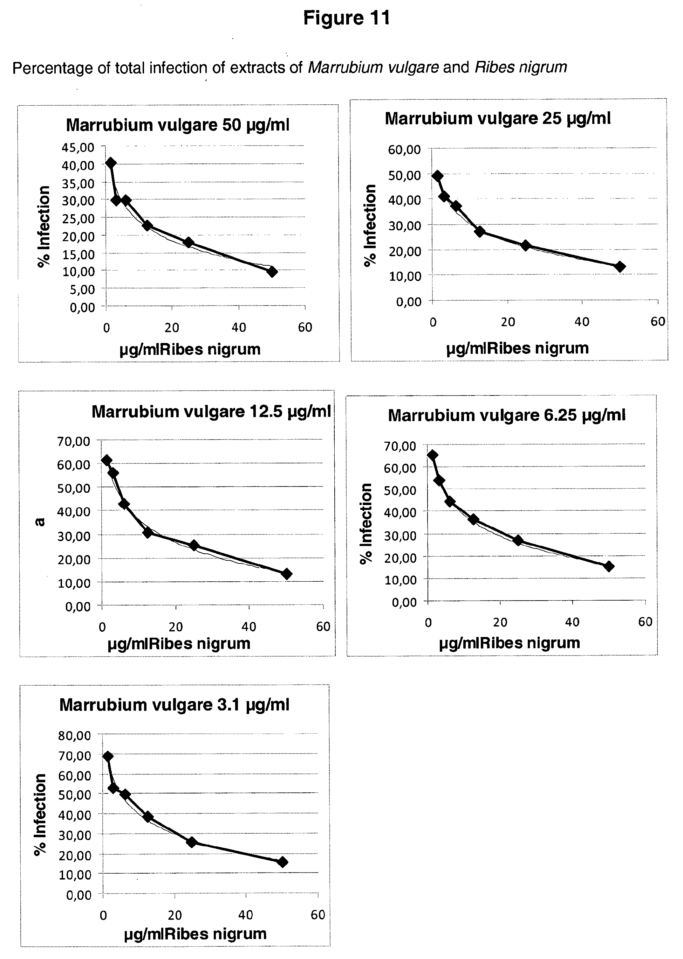

Table 23: Infected cells for combination of *Marrubium vulgare* and *Ribes nigrum* (Figure 11)

| *Ribes nigrum* concentration (μg/mL) | Infected cells in combination with 100 μg/ml of *Marrubium vulgare* (%) | Percent of total infection (%) | Infected cells in combination with 50 μg/ml of *Marrubium vulgare* (%) | Percent of total infection (%) | Infected cells in combination with 25 μg/ml of *Marrubium vulgare* (%) | Percent of total infection (%) |
|---|---|---|---|---|---|---|
| 1.5 | 5.80 | 19.66 | 11.85 | 40.17 | 14.4 | 48.81 |
| 3.1 | 5.60 | 18.98 | 8.8 | 29.83 | 12.1 | 41.02 |
| 6.25 | 4.55 | 15.42 | 8.8 | 29.83 | 11 | 37.29 |
| 12.5 | 3.90 | 13.22 | 6.7 | 22.71 | 7.95 | 26.95 |
| 25 | 3.30 | 11.19 | 5.3 | 17.97 | 6.3 | 21.36 |
| 50 | 1.51 | 5.12 | 2.8 | 9.49 | 3.85 | 13.05 |

Table 24: Infected cells for combination of *Marrubium vulgare* and *Ribes nigrum* (Figure 11)

| *Ribes nigrum* concentration (μg/mL) | Infected cells in combination with 12.5 μg/ml of *Marrubium vulgare* (%) | Percent of total infection (%) | Infected cells in combination with 6.25 μg/ml of *Marrubium vulgare* (%) | Percent of total infection (%) | Infected cells in combination with 3.1 μg/ml of *Marrubium vulgare* (%) | Percent of total infection (%) |
|---|---|---|---|---|---|---|
| 1.5 | 18.15 | 61.53 | 19.25 | 65.25 | 20.35 | 68.98 |

(continued)

| Ribes nigrum concentration (μg/mL) | Infected cells in combination with 12.5 μg/ml of Marrubium vulgare (%) | Percent of total infection (%) | Infected cells in combination with 6.25 μg/ml of Marrubium vulgare (%) | Percent of total infection (%) | Infected cells in combination with 3.1 μg/ml of Marrubium vulgare (%) | Percent of total infection (%) |
|---|---|---|---|---|---|---|
| 3.1 | 16.55 | 56.10 | 15.90 | 53.90 | 15.50 | 52.54 |
| 6.25 | 12.70 | 43.05 | 13.15 | 44.58 | 14.65 | 49.66 |
| 12.5 | 9.10 | 30.85 | 10.70 | 36.27 | 11.40 | 38.64 |
| 25 | 7.50 | 25.42 | 7.95 | 26.95 | 7.60 | 25.76 |
| 50 | 3.80 | 12.88 | 4.40 | 14.92 | 4.55 | 15.42 |

The second, fourth and sixth columns of Table 21 to 24 show the percentages of infected cells of the combinations of *Marrubium vulgare* and *Ribes nigrum* and were taken from Table 16 at the respective concentrations. In the third, fifth and seventh columns the percentages of infected cells based on the virus-only value (29.5%) are given, i.e. 29.5% corresponds to 100% of total infection.

The $IC_{50}$ and $IC_{75}$ values of one of the substances in the mixture are determined from the graphs shown in Figures 10 and 11 at a particular concentration of the other substance in the mixture. From the $IC_{50}$ and $IC_{75}$ values the fractional inhibitory concentration (FIC) indices were calculated. The FIC index is used as a predictor of synergy and indicates whether an observed effect is synergistic or merely additive. A FIC index below 1 indicates a synergistic effect. In particular, the following applies:

FIC < 1: synergistic effect

FIC = 1: additive effect

FIC > 1: antagonistic effect.

The FIC index is calculated as follows:

$$FIC = Ac/Ae + Bc/Be$$

with: Ac = concentration of first substance in mixture at $IC_{50}$ or $IC_{75}$, respectively
Ae = $IC_{50}$ or $IC_{75}$, respectively of first substance alone;
Bc = concentration of second substance in mixture at $IC_{50}$ or $IC_{75}$, respectively
Be = $IC_{50}$ or $IC_{75}$, respectively of second substance alone.

Table 25: $IC_{50}$ and $IC_{75}$ values for combinations of *Marrubium vulgare* and *Ribes nigrum*

| Ribes nigrum concentration (μg/ml) Bc | IC$_{75}$ from Marrubium vulgare in combination with Ribes nigrum (μg/ml) Ac | FIC | IC$_{50}$ from Marrubium vulgare in combination with Ribes nigrum (μg/ml) Ac | FIC |
|---|---|---|---|---|
| 1.5 | | | 18.6 | 0.87 |
| 3.1 | 79 | 0.85 | 6.4 | 0.60 |
| 6.25 | 63.4 | 0.78 | 4.1 | 0.89 |
| 12.5 | 27.4 | 0.60 | | |
| 25 | 7.6 | 0.74 | | |
| *Ribes nigrum:* $IC_{50}$ = 8.5 μg/ml, $IC_{75}$ = 37.3 μg/ml; Be <br> *Marrubium vulgare*: $IC_{50}$ = 26.8 μg/ml, $IC_{75}$ =103.2 μg/ml; Ae | | | | |

Table 26: IC$_{50}$ and IC$_{75}$ values for combinations of *Marrubium vulgare* and *Ribes nigrum*

| *Marrubium vulgare* concentration (μg/ml) Bc | IC$_{75}$ from *Ribes nigrum* in combination with *Marrubium vulgare* (μg/ml) Ac | FIC | IC$_{50}$ from *Ribes nigrum* in combination with *Marrubium vulgare* (μg/ml) Ac | FIC |
|---|---|---|---|---|
| 3.1 | 27.6 | 0.77 | 5 | 0.70 |
| 6.25 | 26.3 | 0.77 | 4.4 | 0.75 |
| 12.5 | 22.4 | 0.72 | 3.8 | 0.91 |
| 25 | 16.6 | 0.69 | | |
| 50 | 8.76 | 0.72 | | |

*Ribes nigrum:* IC$_{50}$ = 8.5 μg/ml, IC$_{75}$ = 37.3 μg/ml; Ae
*Marrubium vulgare*: IC$_{50}$ = 26.8 μg/ml, IC$_{75}$ =103.2 μg/ml; Be

As can be seen from Tables 25 and 26, the FIC indices are all below 1, i.e. the combination of *Marrubium vulgare* and *Ribes nigrum* shows a synergistic effect.

Accordingly, the percentages of total infection for combinations of *Marrubium vulgare* and *Cistus incanus* were determined. The virus-only value of 22.6% corresponds to 100% of total infection.

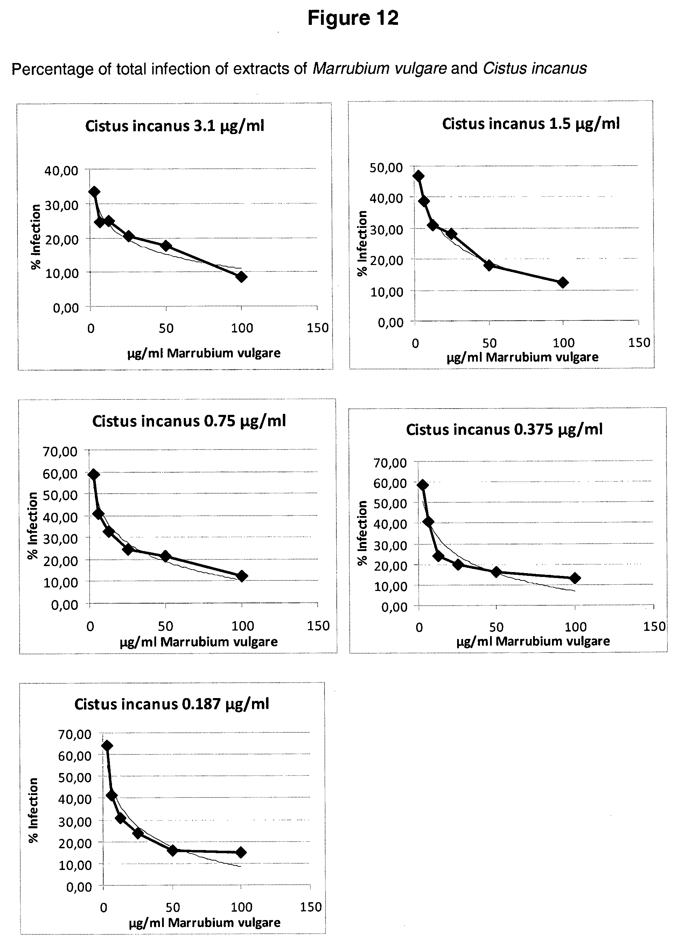

Table 27: Infected cells for combination of *Marrubium vulgare* and *Cistus incanus* (Figure 12)

| *Marrubium vulgare* concentration (μg/mL) | Infected cells in combination with 6.25 μg/ml of *Cistus incanus* (%) | Percent of total infection (%) | Infected cells in combination with 3.1 μg/ml of *Cistus incanus* (%) | Percent of total infection (%) | Infected cells in combination with 1.5 μg/ml of *Cistus incanus* (%) | Percent of total infection (%) |
|---|---|---|---|---|---|---|
| 3.1 | 5.70 | 25.22 | 7.52 | 33.27 | 10.56 | 46.73 |
| 6.25 | 4.20 | 18.58 | 5.54 | 24.51 | 8.76 | 38.76 |
| 12.5 | 4.72 | 20.88 | 5.6 | 24.78 | 7 | 30.97 |
| 25 | 4.59 | 20.31 | 4.61 | 20.40 | 6.4 | 28.32 |
| 50 | 3.46 | 15.31 | 4.00 | 17.70 | 4.03 | 17.83 |
| 100 | 1.47 | 6.50 | 1.92 | 8.50 | 2.78 | 12.30 |

Table 28: Infected cells for combination of *Marrubium vulgare* and *Cistus incanus* (Figure 12)

| *Marrubium vulgare* concentration (μg/mL) | Infected cells in combination with 0.75 μg/ml of *Cistus incanus* (%) | Percent of total infection (%) | Infected cells in combination with 0.375 μg/ml of *Cistus incanus* (%) | Percent of total infection (%) | Infected cells in combination with 0.187 μg/ml of *Cistus incanus* (%) | Percent of total infection (%) |
|---|---|---|---|---|---|---|
| 3.1 | 13.30 | 58.85 | 13.26 | 58.67 | 14.45 | 63.94 |
| 6.25 | 9.25 | 40.93 | 9.17 | 40.58 | 9.35 | 41.37 |
| 12.5 | 7.38 | 32.65 | 5.38 | 23.81 | 6.95 | 30.75 |
| 25 | 5.59 | 24.73 | 4.46 | 19.73 | 5.40 | 23.89 |
| 50 | 4.90 | 21.68 | 3.63 | 16.06 | 3.60 | 15.93 |
| 100 | 2.73 | 12.08 | 2.98 | 13.19 | 3.36 | 14.87 |

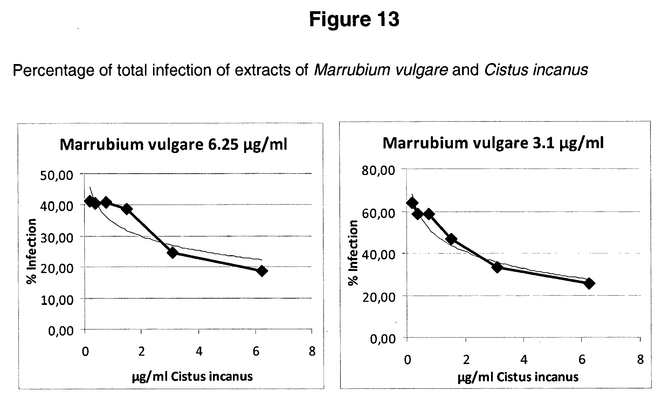

Table 29: Infected cells for combination of *Marrubium vulgare* and *Cistus incanus* (Figure 13)

| *Cistus incanus* concentration (Ng/mL) | Infected cells in combination with 100 µg/ml of *Marrubium vulgare* (%) | Percent of total infection (%) | Infected cells in combination with 50 µg/ml of *Marrubium vulgare* (%) | Percent of total infection (%) | Infected cells in combination with 25 µg/ml of *Marrubium vulgare* (%) | Percent of total infection (%) |
|---|---|---|---|---|---|---|
| 0.187 | 3.36 | 14.87 | 3.6 | 15.93 | 5.4 | 23.89 |
| 0.375 | 2.98 | 13.19 | 3.63 | 16.06 | 4.46 | 19.73 |
| 0.75 | 2.73 | 12.08 | 4.9 | 21.68 | 5.59 | 24.73 |
| 1.5 | 2.78 | 12.30 | 4.03 | 17.83 | 6.4 | 28.32 |
| 3.1 | 1.92 | 8.50 | 4 | 17.70 | 4.61 | 20.40 |
| 6.25 | 1.47 | 6.50 | 3.46 | 15.31 | 4.59 | 20.31 |

Table 30: Infected cells for combination of *Marrubium vulgare* and *Cistus incanus* (Figure 13)

| *Cistus incanus* concentration (µg/mL) | Infected cells in combination with 12.5 µg/ml of *Marrubium vulgare* (%) | Percent of total infection (%) | Infected cells in combination with 6.25 µg/ml of *Marrubium vulgare* (%) | Percent of total infection (%) | Infected cells in combination with 3.1 µg/ml of *Marrubium vulgare* (%) | Percent of total infection (%) |
|---|---|---|---|---|---|---|
| 0.187 | 6.95 | 30.75 | 9.35 | 41.37 | 14.45 | 63.94 |
| 0.375 | 5.38 | 23.81 | 9.17 | 40.58 | 13.29 | 58.81 |
| 0.75 | 7.38 | 32.65 | 9.25 | 40.93 | 13.30 | 58.85 |
| 1.5 | 7.00 | 30.97 | 8.76 | 38.76 | 10.56 | 46.73 |
| 3.1 | 5.60 | 24.78 | 5.54 | 24.51 | 7.52 | 33.27 |
| 6.25 | 4.72 | 20.88 | 4.20 | 18.58 | 5.70 | 25.22 |

The second, fourth and sixth columns of Tables 27 to 30 show the percentages of infected cells of the combinations of *Marrubium vulgare* and *Cistus incanus* and were taken from Table 17 at the respective concentrations. In the third, fifth and seventh columns the percentages of infected cells based on the virus-only value (22.6%) are given, i.e. 22.6% corresponds to 100% of total infection.

The following $IC_{50}$ and $IC_{75}$ values were determined in the same manner as indicated above from the graphs shown in Figures 12 and 13 and the FIC indices were calculated.

Table 31: $IC_{50}$ and $IC_{75}$ values for combinations of *Marrubium vulgare* and *Cistus incanus*

| *Cistus incanus* concentration (µg/ml) Bc | $IC_{75}$ from *Marrubium vulgare* in combination with *Cistus incanus* (µg/ml) Ac | FIC | $IC_{50}$ from *Marrubium vulgare* in combination with *Cistus incanus* (µg/ml) Ac | FIC |
|---|---|---|---|---|
| 0.187 | 29.1 | 0.48 | 4.6 | 0.32 |
| 0.375 | 23.6 | 0.42 | 3.2 | 0.33 |
| 0.75 | 30.7 | 0.58 | 4 | 0.52 |
| 1.5 | 26.9 | 0.61 | | |
| 3.1 | 10 | 0.54 | | |
| *Cistus incanus:* $IC_{50}$ = 2.37 µg/ml, $IC_{75}$ = 7.98 µg/ml; Be  *Marrubium vulgare:* $IC_{50}$ = 18.7 µg/ml, $IC_{75}$ = 62.8 µg/ml; Ae | | | | |

Table 32: $IC_{50}$ and $IC_{75}$ values for combinations of *Marrubium vulgare* and *Cistus incanus*

| *Marrubium vulgare* concentration (µg/ml) Bc | $IC_{75}$ *from Cistus incanus* in combination with *Marrubium vulgare* (µg/ml) Ac | FIC | $IC_{50}$ from *Cistus incanus* in combination with *Marrubium vulgare* (µg/ml) Ac | FIC |
|---|---|---|---|---|
| 3.1 | | | 0.886 | 0.54 |
| 6.25 | 4.17 | 0.62 | | |
| *Cistus incanus:* $IC_{50}$ = 2.37 µg/ml, $IC_{75}$ = 7.98 µg/ml; Ae <br> *Marrubium vulgare:* $IC_{50}$ = 18.7 µg/ml, $IC_{75}$ = 62.8 µg/ml; Be | | | | |

As can be seen from Tables 31 and 32, the FIC indices are all below 1, i.e. the combination of *Marrubium vulgare* and *Cistus incanus* shows a synergistic effect.

**Claims**

1. Composition for use in the prophylaxis and/or treatment of viral infections caused by retroviruses, comprising a concentrate or an extract of a plant of the genus marrubium in combination with at least one concentrate or extract of another plant selected from *Ribes nigrum, Cistus incanus,* and *Borago officinalis.*

2. The composition for use according to claim 1, wherein the concentrate or extract is from the aboveground parts of the plant.

3. The composition for use according to claim 1 or 2, wherein the concentrate or extract of a plant of the genus marrubium is from *Marrubium vulgare.*

4. The composition for use according to any one of claims 1 to 3, comprising two or more concentrates or extracts of the other plant.

5. The composition for use according to any one of claims 1 to 4, wherein the viral infection is caused by exogenous retroviruses.

6. The composition for use according to claim 5, wherein the exogenous retroviruses are HIV-1 or HIV-2.

7. The composition for use according to any one of claims 1 to 6, wherein the composition is in liquid, dry or semi-solid form.

8. The composition for use according to any one of claims 1 to 7, wherein the extract is an aqueous extract or an alcoholic extract.

9. The composition for use according to any one of claims 1 to 8, wherein the composition is administered orally, topically, rectally, or vaginally.

10. The composition for use according to any one of claims 1 to 9, wherein the composition is in the form of an aerosol, mouthwash, or mouth spray.

11. The composition for use according to any one of claims 1 to 9, wherein the composition is in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution or plant juice.

**Patentansprüche**

1. Zusammensetzung zur Verwendung in der Prophylaxe und/oder Behandlung von Virusinfektionen, die durch Retroviren verursacht werden, umfassend ein Konzentrat oder einen Extrakt von einer Pflanze der Gattung Marrubium in Kombination mit mindestens einem Konzentrat oder Extrakt einer anderen Pflanze, ausgewählt aus *Ribes nigrum, Cistus incanus* und *Borago officinalis.*

2. Zusammensetzung zur Verwendung nach Anspruch 1, wobei das Konzentrat oder der Extrakt von den oberirdischen Teilen der Pflanze ist.

3. Zusammensetzung zur Verwendung nach Anspruch 1 oder 2, wobei das Konzentrat oder der Extrakt von einer Pflanze der Gattung Marrubium von *Marrubium vulgare* ist.

4. Zusammensetzung zur Verwendung nach einem der Ansprüche 1 bis 3, umfassend zwei oder mehr Konzentrate oder Extrakte der anderen Pflanze.

5. Zusammensetzung zur Verwendung nach einem der Ansprüche 1 bis 4, wobei die Virusinfektion durch exogene Retroviren verursacht wird.

6. Zusammensetzung zur Verwendung nach Anspruch 5, wobei die exogenen Retroviren HIV-1 oder HIV-2 sind.

7. Zusammensetzung zur Verwendung nach einem der Ansprüche 1 bis 6, wobei die Zusammensetzung in flüssiger, trockener oder halbfester Form ist.

8. Zusammensetzung zur Verwendung nach einem der Ansprüche 1 bis 7, wobei der Extrakt ein wässriger Extrakt oder alkoholischer Extrakt ist.

9. Zusammensetzung zur Verwendung nach einem der Ansprüche 1 bis 8, wobei die Zusammensetzung oral, topisch, rektal oder vaginal verabreicht wird.

10. Zusammensetzung zur Verwendung nach einem der Ansprüche 1 bis 9, wobei die Zusammensetzung in der Form eines Aerosols, Mundwassers oder Mundsprays ist.

11. Zusammensetzung zur Verwendung nach einem der Ansprüche 1 bis 9, wobei die Zusammensetzung in der Form einer Tablette, einer beschichteten Tablette, einer Brausetablette, einer Kapsel, eines Pulvers, eines Granulats, einer zuckerbeschichteten Tablette, einer Lutschtablette, einer Pille, einer Ampulle, eines Drops, eines Zäpfchens, einer Emulsion, einer Salbe, eines Gels, einer Tinktur, einer Paste, einer Creme, einer feuchten Kompresse, einer Gurgellösung oder eines Pflanzensafts ist.


**Revendications**

1. Composition à utiliser dans la prophylaxie et/ou le traitement d'infections virales causées par des rétrovirus, comprenant un concentré ou un extrait d'une plante du genre *Marrubium* en combinaison avec au moins un concentré ou extrait d'une autre plante sélectionnée parmi *Ribes nigrum, Cistus incanus,* et *Borago officinalis.*

2. Composition à utiliser selon la revendication 1, dans laquelle le concentré ou l'extrait provient des parties aériennes de la plante.

3. Composition à utiliser selon la revendication 1 ou 2, dans laquelle le concentré ou l'extrait d'une plante du genre *Marrubium* provient de *Marrubium vulgare.*

4. Composition à utiliser selon l'une quelconque des revendications 1 à 3, comprenant deux ou plusieurs concentrés ou extraits de l'autre plante.

5. Composition à utiliser selon l'une quelconque des revendications 1 à 4, dans laquelle l'infection virale est provoquée par des rétrovirus exogènes.

6. Composition à utiliser selon la revendication 5, dans laquelle les rétrovirus exogènes sont le VIH-1 ou le VIH-2.

7. Composition à utiliser selon l'une quelconque des revendications 1 à 6, dans laquelle la composition se présente sous une forme liquide, sèche ou semi-solide.

8. Composition à utiliser selon l'une quelconque des revendications 1 à 7, dans laquelle l'extrait est un extrait aqueux ou un extrait alcoolique.

9. Composition à utiliser selon l'une quelconque des revendications 1 à 8, dans laquelle la composition est administrée par voie orale, topique, rectale, ou vaginale.

10. Composition à utiliser selon l'une quelconque des revendications 1 à 9, dans laquelle la composition se présente sous la forme d'un aérosol, d'un bain de bouche, ou d'une pulvérisation buccale.

11. Composition à utiliser selon l'une quelconque des revendications 1 à 9, dans laquelle la composition se présente sous la forme d'un comprimé, d'un comprimé enrobé, d'un comprimé effervescent, d'une capsule, d'une poudre, d'un granulé, d'un comprimé enrobé de sucre, d'une dragée, d'une pilule, d'une ampoule, d'une goutte, d'un suppositoire, d'une émulsion, d'un onguent, d'un gel, d'une teinture, d'une pâte, d'une crème, d'une compresse humide, d'une solution pour gargarisme ou d'un jus de plante.

# Figure 1

Antiviral activity of an extract of *Marrubium vulgare* and *Ribes nigrum* against HIV-1 (single extracts and combination)

LU: luminescence units

# Figure 2

Antiviral activity of an extract of *Marrubium vulgare* and *Cistus incanus* against HIV-1 (single extracts and combination)

LU: luminescence units

# Figure 3

Figure 3: Antiviral activity of an extract of *Marrubium vulgare* and *Borago officinalis* against HIV-1 (single extracts and combination)

LU: luminescence units

## Figure 4

Cytotoxicity assay of a combination of an extract of *Marrubium vulgare* and *Borago officinalis*

**Marrubium vulgare/Borago officinalis**

LU: luminescence units

# Figure 5

Cytotoxicity assay of an extract of the twigs and leaves of *Marrubium vulgare* (082), *Ribes nigrum* (049), *Cistus incanus* (050), *Marrubium vulgare* and *Ribes nigrum* (049/082), and *Marrubium vulgare* and *Cistus incanus* (050/082)

LU: luminescence units

## Figure 6

Percentage of total infection of extract of *Ribes nigrum*

## Figure 7

Percentage of total infection of extract of *Marrubium vulgare*

# Figure 8

Percentage of total infection of extract of *Cistus incanus*

# Figure 9

Percentage of total infection of extract of *Marrubium vulgare*

# Figure 10

Percentage of total infection of extracts of *Marrubium vulgare* and *Ribes nigrum*

# Figure 11

Percentage of total infection of extracts of *Marrubium vulgare* and *Ribes nigrum*

# Figure 12

Percentage of total infection of extracts of *Marrubium vulgare* and *Cistus incanus*

# Figure 13

Percentage of total infection of extracts of *Marrubium vulgare* and *Cistus incanus*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KASHMAN et al.** *Journal of Medicinal Chemistry,* 1992, vol. 35 (15), 2735-2743
- **DHARMARATNE et al.** *Planta Medica,* 1998, vol. 64 (5), 460-461
- **HU et al.** *Journal of Natural Products,* 1994, vol. 57 (1), 42-51
- **SILVIA GEUENICH.** Untersuchungen zum Patho-genitätspotential von HIV-1 Primärisolaten und mechanistische Analysen zur antiviralen Aktivitat wassriger Extrakte und Flavonoide aus Lamiaceen. *Inaugural Dissertation, Heidelberg,* 2008

- Hager's Handbuch der pharmazeutischen Praxis. Springer-Verlag, 1993, 777-782
- Hager's Handbuch der pharmazeutischen Praxis. Springer-Verlag, 1993, 466-474
- Hager's Handbuch der pharmazeutischen Praxis. Springer-Verlag, 1993, 528-532